Europäisches Patentamt
European Patent Office
Office européen des brevets

(11) Publication number: **0 094 727**
**A2**

# EUROPEAN PATENT APPLICATION

(21) Application number: 83200690.2

(22) Date of filing: 16.05.83

(51) Int. Cl.[3]: C 07 D 213/38
C 07 D 231/12, C 07 D 233/64
C 07 D 233/68, C 07 D 233/84
C 07 D 233/95, C 07 D 239/26
C 07 D 249/08, C 07 D 263/32
C 07 D 271/06, C 07 D 277/28

(30) Priority: 18.05.82 IT 2133182

(43) Date of publication of application:
23.11.83 Bulletin 83/47

(84) Designated Contracting States:
AT BE CH DE FR GB IT LI LU NL SE

(71) Applicant: ISTITUTO DE ANGELI S.p.A.
Via Serio, 15
I-20139 Milano(IT)

(72) Inventor: Cereda, Enzo
Via Romagnolo 2A
I-15057 Tortona (Alessandria)(IT)

(72) Inventor: Bietti, Giuseppe
Via Lario 31
I-20159 Milan(IT)

(72) Inventor: Donetti, Arturo
Viale Romagna 4
I-20133 Milan(IT)

(72) Inventor: Del Soldato, Piero
Via E. Toti 22
I-20052 Monza Milan(IT)

(72) Inventor: Giachetti, Antonio
Via Guerrini 3
I-20133 Milan(IT)

(72) Inventor: Pagani, Ferdinando
Via Morigiola 18
I-20050 Verano Brianza Milan(IT)

(74) Representative: Appoloni, Romano et al,
ING. BARZANO' & ZANARDO MILANO S.p.A. Via
Borgonuovo 10
I-20121 Milano(IT)

(54) **Substituted heterocyclyl phenylformamidines, processes for their preparation and their pharmaceutical use.**

(57) Object of the present invention are new pharmacologically active substituted heterocyclylphenylformamidines of formula

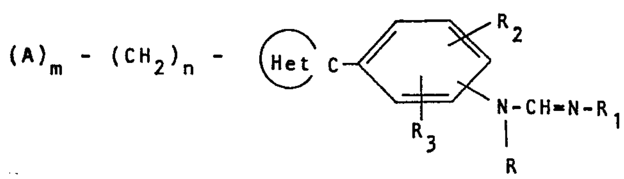

(I)

in which A represents a hydrogen atom, an amino group which may be substituted by one or two alkyl groups having 1 to 3 carbon atoms or a saturated heterocyclic ring, R represents a hydrogen atom or a methyl group, $R_1$ represents a linear or branched alkyl group which may contain 1 or 2 heteroatoms such as a sulfur, an oxygen or nitrogen atom or may form a substituted or an unsubstituted cyclic ring, an alkenyl group, an alkynyl group, a cycloalkyl group or a phenyl group, $R_2$ and $R_3$ which may be the same or different, represent a hydrogen atom, an alkyl or alkoxy group having 1 to 3 carbon atom or a halogen atom, m and n represent 0 or 1, Het represents a substituted or unsubstituted heterocyclic ring containing from 1 to 3 heteroatoms (except the imidazole ring when $R_1$ is a linear or branched alkyl group not containing heteroatoms and $R_2$ and $R_3$ are both hydrogen atoms), tautomers thereof and acid addition salts of the aforesaid compounds. Processes for preparation of the compounds of formula (I) and their intermediates as well as pharmaceutical compositions containing them are also object of this invention.

The new compounds are $H_2$-receptor blocking agents, inhibit gastric acid secretion, and are useful antiulcer agents.

EP 0 094 727 A2

0094727

The present invention relates to new substituted heterocyclyl-phenylformamidines, to processes for their preparation and to pharmaceutical compositions containing them. The new compounds are $H_2$-receptor blocking agents and may be used as gastric acid secretion inhibitors and antiulcer agents.

It is known that classical antihistamines, such as mepyramine, are capable to antagonise some effects of histamine mediated by $H_1$-receptors. However, these compounds have no effect on gastric acid secretion which is instead affected by other antihistaminic agents defined by Black et al. (Nature 236, 335, 1972) as histamine $H_2$-receptor antagonists. This has indicated that another kind of receptors ($H_2$) already preconized by Ash and Schild (Brit. J. Pharmacol. Chem. Ther. 1966, 27, 427-39), is involved in the gastric secretory response which is not blocked by the conventional antihistamines of the $H_1$-type.

Example of $H_2$-receptor antagonists capable of antagonizing gastric acid secretion include burimamide, metiamide, and cimetidine. More recently, new $H_2$-antagonists, such as ranitidine (Bradshaw, et al., Brit. J. Pharmacol. 66, 464P, 1979), tiotidine (P.O. Jellin, Life Sci. 25, 2001, 1979) and BL 6341 (Cavanagh et al., Fed. Proc., 40, 2652, 1981) have been found out. These compounds are effective $H_2$- blockers capable of antagonizing gastric acid secretion at a greater extent than cimetidine and congeners.

In our European Pat.Applns.N°81201192.2, N°83200388.3 we have described new classes of histamine $H_2$-antagonists, namely imidazolyl-phenylamidines and guanidinoheterocyclylphenylamidines, which are potent $H_2$-blockers and active antagonists of gastric acid secretion. The new compounds do not resemble the

so far known $H_2$-antagonists, such as cimetidine, ranitidine, etc., and are characterized by a phenylformamidino groupment bearing variously substituted imidazolyl and guanidinoheterocyclyl rings.

Object of the present invention are heterocyclylphenylformamidines having the following general formula

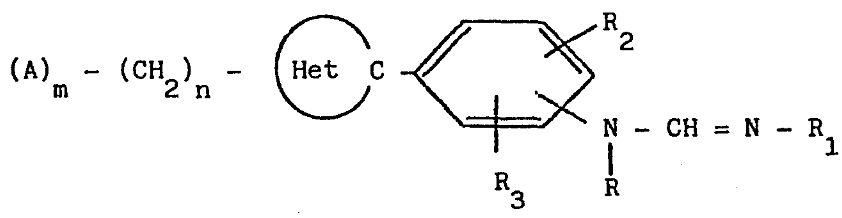 (I)

in which A represents a hydrogen atom, an amino group which may be substituted by one or two alkyl groups having 1 to 3 carbon atoms or a saturated heterocylic ring, R represents a hydrogen atom or a methyl group, $R_1$ represents a linear or branched alkyl group which may contain 1 or 2 heteroatoms such as a sulfur, an oxygen or nitrogen atom or may form a substituted or an unsubstituted cyclic ring, an alkenyl group, an alkynyl group, a cycloalkyl group or a phenyl group, $R_2$ and $R_3$ which may be the same or different, represent a hydrogen atom, an alkyl or alkoxy group having 1 to 3 carbon atoms or a halogen atom, m and n represent 0 or 1, Het represents a substituted or unsubstituted heterocyclic ring containing from 1 to 3 heteroatoms (except the imidazole ring when $R_1$ is a linear or branched alkyl group not containing heteroatoms, and $R_2$ and $R_3$ are both hydrogen atoms), tautomers thereof and acid addition salts of the aforesaid compounds.

For pharmaceutical use the acid addition salts referred to the above will be physiologically compatible acid addition salts. The term "acid addition salts" includes salts with inorganic or organic acids. Suitable physiologically compatible acid addition salts include, for example, salts formed with maleic, citric, hydrochloric, tartaric, hydrobromic, fumaric, nitric or sulphuric acid.

It is to be understood that although the double bond in the formamidine radical has been inserted in a particular position, other tautomeric forms are possible when R is a hydrogen atom, and that in the heterocyclic ring different tautomeric forms are also possible. The present invention includes such tautomeric forms within its scope, both in terms of the compounds of the invention and in terms of the manufacturing processes.

When in the compounds of formula (I) A represents a saturated heterocyclic group this may be a 5- or 6-membered ring which may contain a further heteroatom, e.g. piperidine, morpholine or pyrrolidine; when $R_1$ represents a linear or branched alkyl group it may be an alkyl group containing from 1 to 8 carbon atoms which may contain an oxygen atom, e.g. hydroxypropyl, hydroxybutyl, mono-, di-methoxyethyl, methoxypropyl group, a sulfur atom, e.g. methyltioethyl, ethylthioethyl group, a nitrogen atom, e.g. cyanoethyl, dimethylaminopropyl group, an oxygen and sulfur atom together, e.g. ethoxymethylthioethyl group, or may form a substituted or unsubstituted cyclic ring, such as a 5-membered ring, e.g. dimethyldioxolylmethyl or hydroxymethyldioxolylmethyl; when $R_1$ represents an alkenyl group it may be an alkenyl group containing from 3 to 5 carbon

atoms; when $R_1$ represents an alkynyl group it may be an alkynyl group containing 3 or 4 carbon atoms; when $R_1$ represents a cycloalkyl group it may contain from 3 to 6 carbon atoms; when $R_2$ and/or $R_3$ represent a halogen atom these may be a bromine, chlorine or fluorine atom; when Het represents a substituted or unsubstituted heterocyclic ring containing from 1 to 3 heteroatoms it may be a 5- or 6-membered ring, e.g. imidazole, nitroimidazole, bromoimidazole, N-methyl-imidazole, oxazole, 1,2,4--oxadiazole, pyrazole, furan, thiophene, thiazole, 1,2,4-triazole, imidazoline, pyrimidine, pyridine, pyrimidone ring.

In the above mentioned formula (I) the formamidine radical may be in the ortho, meta or para position of the benzene ring with respect to the Het group, and the group $R_2$ and/or $R_3$ are in any position in the benzene ring.

Preferred compounds according to the present invention include those wherein the formamidine radical is in the para position with respect to the heterocyclic ring, m and n are 0, R represents a hydrogen atom, $R_1$ is propyl, isopropyl, butyl, sec-butyl, isobutyl, neopentyl, allyl, methoxyethyl, dimethoxyethyl, hydroxypropyl, hydroxybutyl, methylthioethyl, ethylthioethyl, cyanoethyl or ethoxymethylthioethyl group, $R_2$ and/or $R_3$ represent a hydrogen atom, a methyl, methoxy group, a bromine, chlorine or fluorine atom, Het is 3-pyrazolyl, 3-(1,2,4-triazolyl) or 4-imidazolyl group and their physiologically compatible acid addition salts. Such compounds generally have better activity and are therefore preferred as antisecretory-antiulcer agents and for the treatment of disorders of the gastrointestinal tract.

The compounds of general formula (I) may, for example, be prepared by the following processes which constitute further features of the invention.

Process A. Reaction of a N,N'-disubstituted formamidine of formula (II)

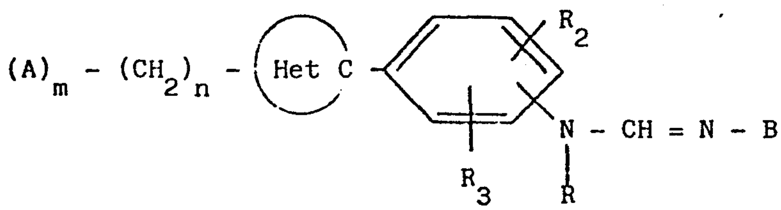

(II)

(wherein R, $R_2$, $R_3$, A, Het, m and n are as hereinbefore defined and B represents a suitable leaving group, such as cyano) with an amine of formula (III)

$$H_2N - R_1 \qquad (III)$$

wherein $R_1$ is as hereinbefore defined.

The reaction may conveniently be performed in the presence of water or of an inert aqueous organic solvent, for example, lower alcohols, such as methanol or ethanol, formamide, dimethylformamide, dioxane or acetonitrile. The reaction is generally carried out at a temperature from 10° to 50°C, preferably at room temperature.

The compound of formula (II) used as starting material in the above process may be prepared by methods that are known in the literature, for example, by reacting an amine of formula (IV)

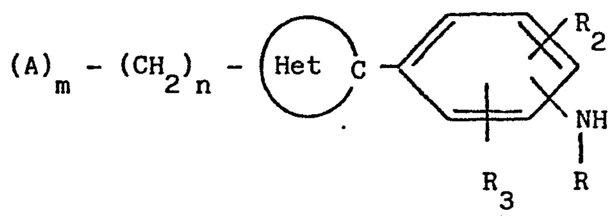

(IV)

(wherein R, $R_2$, $R_3$, Het, A, m and n are as hereinbefore defined) with a N-substituted alkylimidate of formula (V)

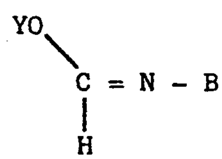

(V)

in which B is as hereinbefore defined and Y is a lower alkyl group, such as methyl or ethyl. The reaction may generally be carried out in the presence of a suitable inert organic solvent, such as a lower alcohol, ethers, ethylacetate, acetonitrile or dioxane or also without solvent at a temperature from 20° to 80°C, preferably at room temperature.

Optionally the compounds of general formula (II) may be prepared in a single step by reacting an amine of formula (IV) with cyanamide in the presence of a compound of formula (VI)

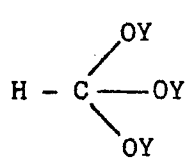

(VI)

in which Y is as hereinbefore defined. The reaction is carried out at a temperature from 50° to 150°C.

When in the compounds of general formula (II) m and n are O, R, $R_2$ and $R_3$ are hydrogen atoms and Het represents 5-nitroimidazol--4-yl or 2-mercaptoimidazol-4-yl, the reaction is performed by reacting the sodium salt of the compounds of formula (IV) with the compound of formula (V) as above described.

Process B. For the preparation of compounds of general formula (I) in which $R_1$ represents a phenyl group: reaction of an amine of formula (IV) with a N-phenyl alkylimidate of formula (VII)

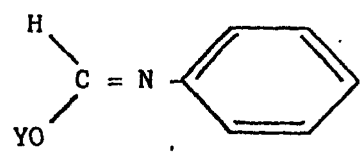

(VII)

in which Y is as hereinbefore defined. The reaction may generally be carried out at a temperature from 0° to 100°C, preferably at room temperature. The reaction is preferably performed either in the absence of any solvent or in the presence of an inert organic solvent, for example, alcohols having 1 to 3 carbon atoms such as methanol or ethanol, halogenated hydrocarbons such as dichloromethane, dioxane or acetone.

The compounds of general formula (I) prepared according to the processes A and B may optionally be converted with inorganic or organic acids into physiologically compatible acid addition salts, for example by conventional methods such as by reacting the

compounds as bases with a solution of the corresponding acid in a suitable solvent or by adding directly the acid solution into the reaction mixture, according to the process A, without isolation of the compound as a base.

Particularly preferred acids include for example hydrochloric, sulphuric, maleic and fumaric acid. The compounds of general formula (I) and their physiologically compatible acid addition salts are $H_2$--receptor blocking agents which inhibit the gastric acid secretion.

Particularly preferred compounds according to the present invention are the following:

- N-(2-Methoxy ethyl)-N'-[4-(imidazol-4-yl)-phenyl] formamidine (Compound 1)

- N-(2-Hydroxy propyl)-N'-[4-(imidazol-4-yl)-phenyl] formamidine (Compound 2)

- N-(2-Methyl thio ethyl)-N'-[4-(imidazol-4-yl)-phenyl] formamidine (Compound 3)

- N-(2-Ethoxymethyl thio ethyl)-N'-[4-(imidazol-4-yl)-phenyl] formamidine (Compound 76)

- N-(2-Cyano ethyl)-N'-[4-(imidazol-4-yl)-phenyl] formamidine (Compound 11)

- N-n-Butyl-N'-[4-(imidazol-4-yl)-2-methyl-phenyl] formamidine (Compound 12)

- N-Allyl-N'-[4-(imidazol-4-yl)-2-methyl-phenyl] formamidine (Compound 13)

- N-Propyl-N'-/¯4-(imidazol-4-yl)-2-methoxy-phenyl_7 formamidine (Compound 14)

- N-Isopropyl-N'-/¯4-(pyrazol-3-yl)-phenyl_7 formamidine (Compound 23)

- N-Propyl-N'-/¯4-(imidazol-4-yl)-2-bromo-phenyl_7 formamidine (Compound 77)

- N-Isopropyl-N'-/¯4-(1.2.4-triazol-3-yl)-phenyl_7 formamidine (Compound 86)

The antagonistic activity of compounds according to the invention on histamine $H_2$-receptors is demonstrated either "in vitro" or "in vivo" by their inhibition of the $H_2$ -dependent biological effects, which include the histamine evoked positive chronotropic effect and the histamine induced gastric secretion of acid respectively.

The inhibition of the positive chronotropic effect has been investigated in isolated guinea pig atria suspended in an organ bath (50 ml) containing oxygenated ($O_2$ : 95% - $CO_2$ : 5%) Krebs - Henseleit solution (pH 7.4) maintained at 32°C. The myocardial preparation, loaded with 1 g isometric tension, is allowed to stabilize 60 min., and myocardial contractions are recorded through an isometric lever connected to a strain-gauge coupler, and the instantaneous rate is monitored with a cardiotachometer, and a heatwriting pen recorder. After two control responses to histamine ($10^{-6}$ $g.ml^{-1}$) the test compounds are added to the bath at the desired final concentration and left for 30 minutes before

the atria are again challenged with histamine. The chronotropic response obtained in the presence of the antagonist is then compared to the control response to histamine and the percent reduction of the histamine $H_2$-evoked response is calculated. The average effective concentration ($EC_{50}$) of the $H_2$ antagonists is also calculated by standard procedure according to Dr. Waud, Analysis of dose-response curves, in "Methods in Pharmacology" vol. 3 Smooth muscle, Ed Daniel E.E. Paton, M., Plenum Press. New York (1975); Ash and Schild, Br. J. Pharmacol. Chemother. 27, 427-439, 1966. The following Table shows the obtained results:

TABLE 1

"In vitro" inhibitory activity in histamine induced tachycardia (guinea pig atria)

| Compound | $EC_{50}$ $10^{-7}$M |
|---|---|
| 1 | 5.88 |
| 2 | 12.7 |
| 3 | 3.27 |
| 76 | 3.20 |
| 11 | 16.0 |
| 12 | 3.09 |
| 13 | 2.94 |
| 14 | 6.00 |
| 78 | 3.50 |
| 23 | 3.90 |
| 86 | 9.95 |
| CIMETIDINE | 34.0 |

The ability of the test compounds to inhibit histamine induced gastric secretion of acid, has been investigated after intravenous or intraduodenal administration in stomach perfused rats, according to Gosh and Schild (Br. J. Pharmacol. Chemother. 13, 54, 1958).

The preparation of the animals in general anaesthesia (urethane, 1 $g.kg^{-1}$ i.p.) and costant temperature, is achieved by inserting and tying in place polyethyl tubes (PE 50) in the oesophagus and in the pyloric-antral region. After the stomach is washed to remove residual of foods, continuous perfusion of the stomach was started with saline, 0.5 $ml.min^{-1}$ (37°C), primed by a Jobling peristaltic pump. After 30 min of perfusion adaptation, the stomach perfusate is collected in 30 min samples, and titrated for acid content, expressed as $\mu$Eq of NaOH 1 N. As control acid output becomes constant intravenous perfusion of histamine ($1mg.kg^{-1}$ $hr^{-1}$) is started and maintained throughout the experimental period. After the acid secretion has reached the steadily higher level, increasing doses of the test compounds are injected intravenously in order to obtain dose response functions. $ED_{50}$ were then calculated by standard procedure.

The results are reported in the following Table:

## TABLE II

"In vivo" antisecretory activity in histamine induced gastric secretion (stomach perfused rat)

| Compound | $ED_{50}$ mg.kg$^{-1}$ (i.v.) * |
|---|---|
| 1 | 0.050 |
| 2 | 0.043 |
| 3 | 0.022 |
| 76 | 0.083 |
| 11 | 0.046 |
| 12 | 0.078 |
| 13 | 0.090 |
| 14 | 0.059 |
| 78 | 0.117 |
| 23 | 0.185 |
| 86 | 0.121 |
| CIMETIDINE | 0.560 |

* The values of activity are expressed taking the compound as a base.

The acute toxicity of the particularly preferred compounds of general formula (I) was approximately determined by oral administration of a single dose to groups of 5 Swiss mice fasting for 18 hours before the experiments. The evaluation of the incidence of mortality was considered after 14 days from the administration. The results are reported in the following Table, in which the values are expressed taking the compound as a base.

TABLE III

| Compound | No. Dead/No. Treated Mice |
|---|---|
| 1 | 0/5 at 500 mg/kg |
| 2 | 3/5 at 500 mg/kg |
| 3 | 0/5 at 500 mg/kg |
| 76 | 1/5 at 500 mg/kg |
| 11 | 0/5 at 500 mg/kg |
| 12 | 4/5 at 500 mg/kg |
| 13 | 3/5 at 500 mg/kg |
| 14 | 4/5 at 500 mg/kg |
| 78 | 4/5 at 500 mg/kg |
| 23 | 4/5 at 500 mg/kg |
| 86 | 4/5 at 500 mg/kg |

0094727

According to a further feature of the present invention there are provided pharmaceutical compositions comprising as active ingredient at least one compound of formula (I), as hereinbefore defined, or a physiologically compatible acid addition salt thereof in association with a pharmaceutical carrier or excipient. For pharmaceutical administration the compounds of general formula (I) and their physiologically compatible acid addition salts may be incorporated into the conventional pharmaceutical preparations in either solid or liquid form. The compositions may, for example, be presented in a form suitable for oral or parenteral administration. Preferred forms include for example, capsules, tablets, coated tables, ampoules or vials containing lyophilized mass of the salt.

The active ingredient may be incorporated in excipients or carriers conventionally used in pharmaceutical compositions such as for example talc, gum arabic, lactose, gelatine, magnesium stearate, corn starch, aqueous or non-aqueous vehicles, polyvinylpirrolidone, mannitol.

The compositions are advantageously formulated at dosage unit, each dosage unit being adapted to supply a fixed dose of the active ingredient. Each dosage unit may conveniently contain from 10 mg to 500 mg and preferably from 20 mg to 150 mg.

The following example illustrate some of the new compounds according to the present invention; these examples are not to be in any way considered limitative of the scope of the invention itself:

Example 1

3-(4-Aminophenyl)-pyrazole

a) A mixture of 4-nitroacetophenone (24.8 g) and N,N'-dimethyl formamide diethyl acetale (33.1 g) was refluxed for 17 hours. 27.4 g of 1-(4-nitrophenyl)-3-N,N-dimethylamino-2-propen-1--one were obtained by crystallizing the residue from diethyl ether, M.p. 148°C.

b) A solution of 1-(4-nitrophenyl)-3-N,N-dimethylamino-2-propen--1-one (27.3 g) and 85% hydrazine hydrate (23.6 g) in ethanol was heated at 60°C for 1 hour. The crystallized solid was filtered to give 18.5 g of 3-(4-nitrophenyl)-pyrazole, M.p. 196-197°C.

c) In a refluxing suspension of 3-(4-nitrophenyl)-pyrazole (18 g) and Ni-Raney (3.3 g) in methanol (190 ml) was added dropwise a solution of 85% hydrazine hydrate (22 g) in methanol (50 ml). The solution was refluxed further for 30 minutes, filtered and evaporated to dryness. The oily residue, after crystalisation from water, furnished 14.5 g of 3-(4-aminophenyl)-pyrazole, M.p. 104°C.

3-(3-Aminophenyl)-pyrazole, M.p. 144-146°C was prepared in a similar manner starting from 3-nitroacetophenone.

Example 2

5-Bromo-4-(4-aminophenyl)-1-H-imidazole

A solution of $Na_2S$ . $8H_2O$ (23.2 g) in methanol (130 ml) and water (40 ml) was dropped into a refluxing suspension of 5-bromo-4-(4--nitrophenyl)-1-H-imidazole (7 g) in methanol (100 ml). After two hours refluxing, the solution was evaporated to dryness, and the residue was taken up into 10% hydrochloric acid and washed several times with ethyl acetate. The crude title compound was obtained as a brown solid neutralizing at pH 6.5 with 10% $NaHCO_3$ the aqueous layer, M.p. 200-201°C.

Example 3

2-Methyl-5-(3-aminophenyl)-oxazole

a) Acetic anhydride (40 g) and sodium acetate (54 g) in water (200 ml) were added to a cold solution of -amino-3-nitroacetophenone hydrochloride (43.6 g) in water and crushed ice (300 g). The temperature was kept under 5° C for an hour. Then 10% hydrochloric acid was added to the solution to give separation of an oily product. This was taken up in ethylacetate, evaporated to dryness to give 15.2 g of the crude N-(3-nitrophenyacyl-)acetamide, M.p. 142-143°C.

b) A solution of N-(3-nitrophenyacyl)-acetamide (15 g) in $POCl_3$ (100 ml) was heated at 80°C for three hours. After evaporation to dryness, the residue was crystalized from water to give 12.2 g of 2-methyl-5-(3-nitrophenyl)-oxazole, M.p. 158-160°C.

c) To a refluxing solution of 2-methyl-5-(3-nitrophenyl)-oxazole (16 g) in methanol (300 ml) was added dropwise a solution of $Na_2S . 8H_2O$ (53 g) in water (370 ml) and methanol (150 ml). The reflux was continued for one hour. The solution was evaporated to dryness, and the residue was taken up into 5% hydrochloric acid; the acid layer was washed several times with ethylacetate, filtered through charcoal and basified to give 8.6 g of 2-methyl-5-(3-aminophenyl)-oxazole, M.p. 110-112°C.

2-Methyl-5-(4-aminophenyl)-oxazole, M.p. 128-129°C was prepared in an analogous manner starting from 2-methyl-5-(4-nitrophenyl)--oxazole.

Example 4

2-(N,N-dimethylamino) methyl-5-(3-aminophenyl)-furane

a) A solution of $SOCl_2$ (10.8 g) in diethylether (40 ml) was added slowly to a suspension of 2-hydroxymethyl-5-(3-nitrophenyl) furane (20 g) in diethylether (450 ml) and pyridine (7.2 g). The reaction was stirred overnight, the solid filtered off and the solution evaporated to dryness to give 20 g of 2-chloromethyl-5-(3-nitrophenyl) furane as a yellow solid, M.p. 95-98°C.

b) A solution of 2-chloromethyl-5-(3-nitrophenyl) furane (14 g) and 33% ethanolic dimethylamine (40 g) in ethanol (150 ml) was heated at 50°C for 20 hours. The residue obtained after evaporation to dryness, was dissolved in ethylacetate and extracted in 10% hydrochloric acid. The aqueous layer was neutralized with 10% NaOH to give an oil which was extracted in ethylacetate

7.6 g of 2-(N,N-dimethylamino)-methyl-5-(3-nitrophenyl) furane as a brown oil, were obtained by evaporating to dryness the organic layer.

2-Morpholinomethyl-5-(3-nitrophenyl furane, brown oil was similarly prepared.

c) 2-(N,N-dimethylamino)-methyl-5-(3-nitrophenyl) furane (14.5 g) in methanol (150 ml) and in the presence of Ni-Raney (1.5 g) was treated with 85% hydrazine hydrate (14 g) in methanol (30 ml), refluxed for 3 hours, filtered and evaporated to dryness. 12.2 g of 2-(N,N-dimethylamino)-methyl-5-(3-aminophenyl) furane were obtained and purified through the hydrochloride salt, M.p. 90-91°C.

2-Morpholinomethyl-5-(3-aminophenyl) furane, grey solid. M.p. 115-7°C was similarly prepared.

Example 5

2-(N,N-dimethylamino)-methyl-4-(4-aminophenyl) furane

a) A solution of 2-chloromethyl-5-(4-nitrophenyl) furane (35 g) and 33% ethanolic dimethylamine (100 g) in ethanol (350 ml) was refluxed for 20 hours and evaporated to dryness. The residue was dissolved into 10% hydrochloric acid and washed with ethylacetate. The oil separated after neutralization of the aqueous layer was extracted in ethylacetate to give, after evaporation, g 30.2 of 2-(N,N-dimethylamino)-methyl-4-(4-nitrophenyl) furane, as a thick reddish cil.

The following compounds were similarly prepared:

2-(N-methylamino)-methyl-5-(4-nitrophenyl) furane, reddish oil.

2-Pyrrolidinemethyl-5-(4-nitrophenyl) furane, brown solid. M.p. 104-106°C

2-(N,N-diethylamino)-methyl-5-(4-nitrophenyl) furane, brown oil.

b) 85% Hydrazine hydrate (25 g) in methanol (50 ml) and water (5 ml) was added dropwise to a refluxing solution of 2-(N,N--dimethylamino)-methyl-5-(4-nitrophenyl)furane (26 g) in methanol (300 ml) in the presence of Ni-Raney (3 g). After two hours refluxing, the solution was evaporated to dryness to give 30 g of 2-(N,N-dimethylamino)-methyl-5-(4-aminophenyl) furane as a yellow oil which could be purified through its oxalate salt.

Similarly were prepared:

2-(N-methylamino)-methyl-5-(4-aminophenyl) furane, M.p. 60--62°C

2-Pyrrolidinomethyl-5-(4-aminophenyl) furane, M.p. 130-132°C

2-(N,N-diethylamino)-methyl-5-(4-aminophenyl) furane, brown oil.

Example 6

2-(N,N-dimethylamino)-methyl-5-(4-aminophenyl) thiophene

a) $NaBH_4$ (2.8 g) was added portionwise to a suspension of 5-(4--nitrophenyl)-2-thiophene carboxaldehyde (16 g) in ethanol (170 ml). The reaction mixture was refluxed for two hours, and evaporated to dryness. The residue was dissolved into ethylacetate and washed with water. The crude 2-hydroxymethyl--5-(4-nitrophenyl) thiophene, M.p. 112-114°C, was obtained by evaporating the organic layer.

Starting from 5-(3-nitrophenyl)-2-thiophene, carboxaldehyde 2-hydroxymethyl-5-(3-nitrophenyl) thiophene was obtained, M.p. 104-106°C.

b) A suspension of 2-hydroxymethyl-5-(4-nitrophenyl) thiophene (16.8 g) in diethylether (370 ml) and pyridine was treated with $SOCl_2$ (8.6 g) in diethylether (30 ml). The reaction mixture was stirred overnight, filtered and evaporated to dryness. The residue, dissolved in ethylacetate washed with water and evaporated, furnished 10.6 g of 2-chloromethyl-5-(4-nitrophenyl) thiophene as a yellow solid, M.p. 133-135°C.

2-Chloromethyl-5-(3-nitrophenyl) thiophene, brown thick oil, was preparated by the same procedure.

c) A solution of 2-chloromethyl-5-(4-nitrophenyl) thiophene (10.8 g) and 33% ethanolic dimethylamine (28 g) in ethanol (100 ml) was refluxed for 20 hours and evaporated to dryness. The residue was taken up in 10% hydrochloric acid washed with ethylacetate and basified to give (9.3 g) of 2-(N,N-dimethylamino)-methyl-5-(4-nitrophenyl) thiophene. Yellow solid, M.p. 58-60°C.

2-Pyrrolidinomethyl-5-(3-nitrophenyl) thiophene, brown oil was prepared in the same manner.

d) 85% Hydrazine hydrate (4.4 g) in methanol (8 ml) and water (1 ml) was dropped into a solution of 2-(N,N-dimethylamino)--methyl-5-(4-nitrophenyl) thiophene (5 g) in methanol (60 ml) in the presence of Ni-Raney (0.6 g). The reaction mixture was refluxed for 2 hours, filtered and evaporated to dryness to give a waxy solid which after crystallization from petroleum ether, furnished 3.2 g of 2-(N,N-dimethylamino)-methyl-5-(4--aminophenyl) thiophene, M.p. 95-96°C.

2-Pyrrolidinomethyl-5-(3-aminophenyl) thiophene, M.p. 129--130°C was similarly prepared.

Example 7

4-(3-Methyl-4-aminophenyl)-1-H-imidazole

a) 4-(3-Methylphenyl)-1-H-imidazole, nitrate salt (31 g) was added portionwise to concentrated sulphuric acid (78 ml) at room temperature. Then the solution was heated at 90°C for 1 hour and cooled. First water (200 ml) was added and then a 17% $Na_2CO_3$ solution till pH was 7.5. The brown solid was filtered and discarged. The solution was strongly basified with 10% NaOH, the yellow solid filtered and dried to give 9.5 g of 4-(3-methyl-4-nitrophenyl)-1-H- imidazole, M.p. 138-140°C.

Similarly was prepared 4-(3-methoxy-4-nitrophenyl)-1-H-imidazole, M.p. 171-173°C.

b) 4-(3-Methyl-4-nitrophenyl)-1-H-imidazole (9.2 g) was allowed to react with 85% hydrazine hydrate in methanol to give 7.9 g of 4-(3-methyl-4-aminophenyl)-1-H-imidazole, M.p. 195-197°C.

A similar reaction furnished 4-(3-methoxy-4-aminophenyl)-1-H--imidazole, M.p. 145°C dec.

Example 8

N-Methyl-N-/‾ 4-(imidazol-4-yl)-phenyl_7 amine

a) A solution of 4-(4-aminophenyl)-1-H-imidazole (10 g) in formic acid (100 ml) was refluxed for 4 hrs and evaporated to dryness. The residue was dissolved in water, filtered on charcoal, and basified to give 10 g of N-/‾4-(imidazol-4-yl)-phenyl_7 formamide, M.p. 108-109°C.

b) A suspension of N-/‾4-(imidazol-4-yl)-phenyl_7 formamide (8 g) in anhydrous THF (80 ml) was slowly dropped into a suspension of $LiAlH_4$ (6.5 g) in anhydrous THF. The reaction mixture was refluxed for 4 hours and cooled. Wet diethylether was added and then the solution was evaporated to dryness. The residue was dissolved into water and ethylacetate. From the organic layer, after usual work up were obtained 4.6 g of N-methyl-N--/‾4-(imidazol-4-yl)-phenyl_7 amine, M.p. 75-76°C.

Example 9

1-Methyl-5-(4-aminophenyl)-imidazole

1-Methyl-5-(4-nitrophenyl)-imidazole (7.3 g) in ethanol (150 ml) was hydrogenated at room temperature and pressure in the presence of 10% C/Pd (0.6 g).When the theoretical amount of hydrogen was taken up, the solution was filtered and evaporated to dryness to give the title compound (4 g), M.p. 119-121°C.

1-Methyl-4-(4-aminophenyl) imidazole, M.p. 171-172°C was similarly prepared.

Example 10

5-Methylamino-3-(4-aminophenyl)-1.2.4-oxadiazole

a) 33% Methylamine (44 g) in ethanol was quickly added to a stirred suspension of 3-(4-nitrophenyl)-5-trichloromethyl-1.2.4-oxadiazole (29 g) in ethanol (300 ml). The reaction mixture was stirred for 4 hours at room temperature and the solid was filtered and dried to give 5-methylamino-3-(4-nitrophenyl)-1.2.4-oxadiazole, M.p. 176-177°C.

5-Methylamino-3-(3-nitrophenyl)-1.2.4-oxadiazole, M.p. 195-196°C was similarly obtained.

b) To a refluxing suspension of 5-methylamino-3-(4-nitrophenyl)-1.2.4-oxadiazole (12 g) in methanol (250 ml) was added dropwise a solution of $Na_2S$ . $8H_2O$ (36 g) in water (100 ml) and methanol (260ml). The reaction was further refluxed, for 1 hour, and

evaporated to dryness. The residue was dissolved into ethylacetate and washed several times with water, and evaporated. 5-Methylamino-3-(4-aminophenyl)-1.2.4-oxadiazole was obtained as a white solid, M.p. 145-147°C.

5-Methylamino-3-(3-aminophenyl)-1.2.4-oxadiazole, M.p. 127-128°C was obtained following the same procedure.

Example 11

2-(N,N-Diethylaminomethyl)-5-(3-aminophenyl)-oxazole

a) α-chloro-N-(3-nitrophenacyl)-acetamide (34 g) was heated for 3 hours at 85°C in the presence of $POCl_3$ (230 g). The solution was cooled and evaporated to dryness to give a residue which after crystallization form water furnished 30 g of 2-chloromethyl-5-(3-nitrophenyl) oxazole, M.p. 104-106°C.

b) A solution of 2-chloromethyl-5-(3-nitrophenyl)-oxazole (15 g) and N,N-diethylamine (23 g) in ethanol (150 ml) was refluxed for 20 hours and evaporated to dryness. The residue was taken up into 10% hydrochloric and, washed with ethylacetate and basified. The oil separated was extracted into ethylacetate to give after evaporation 12.5 g of 2-(N,N-diethylaminomethyl)--5-(3-nitrophenyl)-oxazole as a reddish oil.

2-(N,N-dimethylaminomethyl)-5-(4-nitrophenyl)-oxazole, reddish oil was similarly prepared.

solution was made alkaline with 10% NaOH. The oil which separated was extracted with methylene chloride. The organic solution was evaporated to dryness and the residue was recrystallized from cyclohexane-ligroine to give 40 g of 2-dimethylaminomethyl-4-(4-amino-phenyl)-thiazole, M.p. 84-87°C.

Using the appropriate starting materials, the following thiazole derivatives were prepared in a similar manner:

2-Dimethylaminomethyl-4-(3-amino-phenyl)-thiazole, M.p. 251--253°C (as dihydrochloride)

2-Piperidinomethyl-4-(4-amino-phenyl)-thiazole, M.p. 98-100°C.

Example 13

2-Methylamino-4-(3-amino-phenyl)-thiazole

a) A mixture of ethyl N-/‾3-(2-bromoacetyl)-phenyl_7 carbamate (28.6 g), 1-methyl-thiourea (9 g) in ethanol (75 ml) was refluxed for 4 hours and then cooled.
The precipitate was filtered and washed with cold ethanol to give 34.5 g of ethyl N-/‾3-(2-methylamino-4-thiazolyl)--phenyl_7 carbamate, hydrobromide, M.p. 140-141°C.

b) A mixture of ethyl N-/‾3-(2-methylamino-4-thiazolyl)-phenyl_7 carbamate, hydrobromide (33.5 g) in 10% hydrochloric acid (500 ml) was heated under reflux for 48 hours. After cooling the solution was made alkaline with 10% sodium hydroxide and the precipitate was filtered, washed with water and dried to give 16.5 g of 2-methylamino-4-(3-amino-phenyl)-thiazole, M.p. 118-121°C.

2-Methylamino-4-(4-amino-phenyl)-thiazole, M.p. 178-180°C, was prepared in a similar manner starting from the appropriate carbamate derivative.

Example 14

2-Methyl-4-(3-amino-phenyl)-thiazole

A mixture of 3-amino-phenacyl bromide hydrobromide (11.78 g), thioacetamide (3 g) in dimethylformamide (20 ml) was stirred at room temperature for 24 hours then poured into water (100 ml). The solution was made alkaline with 10% sodium hydroxide and the precipitate was filtered and dried to give 6 g of 2-methyl-4--(3-amino-phenyl)-thiazole, M.p. 134-137°C.

Example 15

4-/(3-bromo-4-amino)-phenyl/-1-H-Imidazole

a) A solution of N-/2-bromo-4-(bromo acetyl)-phenyl/ acetamide (4.6 g) in formamide 7.7 g) and water (0.9 ml) was heated at 140°C for 4 hours. 10% Hydrochloric acid was added to the cold mixture and the solution was washed with èthyl acetate and then neutralized with 10% NaOH. The solid which crystallized out, was filtered and dried to give 2.2 g of 4-/(3--bromo-4-acetamido)-phenyl/-1-H-imidazole, M.p. 215-217°C.

b) A solution of 4-/ (3-bromo-4-acetamido)-phenyl 7-1-H-imidazole (1.8 g) in 6 N hydrochloric acid (7.5 ml) was heated at 80°C for 20 minutes and neutralized with 10% NaOH. The oily product which separated, was extracted into ethyl acetate, washed and evaporated to dryness to give 1.5 g of 4-/ (3-bromo-4-amino)--phenyl 7-1-H-imidazole as a thick reddish oil.

In a similar manner, starting from the suitable intermediates, the following amino derivatives were synthetized:

- 4-/ (3-chloro-4-amino)-phenyl 7-1-H-Imidazole. Yellow oil.

- 4-/ (3.5-dichloro-4-amino)-phenyl 7-1-H-Imidazole.
  M.p. 95-98°C.

- 4-/ 3-chloro-5-bromo-4-amino)-phenyl 7-1-H-Imidazole. Brown oil.

Example 16

5-Pyrrolidinomethyl-3-(3-aminophenyl)-1.2.4-oxadiazole

a) A solution of 5-chloromethyl-3-(3-nitro-phenyl)-1.2.4-oxadiazole (12 g) and pyrrolidine (20 g) in ethanol (200 ml) was refluxed five hours. The solution was evaporated to dryness, taken up into 10% hydrochloric acid and washed with ethylacetate. The aqueous solution was neutralized with 10% NaOH and the oil product, which separated, extracted into ethylacetate. By evaporating to dryness, 5-pyrrolidinomethyl-3-(3-nitro-phenyl)-

-1.2.4-oxadiazole was obtained as a yellow oil (12.1 g).

b) A solution of $SnCl_2 \cdot 2H_2O$ (35 g) in concentrated hydrochloric acid (35 ml) was dropped into a solution of 5-pyrrolidinomethyl-3-(3-nitro-phenyl)-1.2.4-oxadiazole (11 g) in ethanol (110 ml). When the exothermic reaction subsided the reaction mixture was further heated at 50°C for 30 minutes. The ethanol was distilled at reduced pressure and the acid solution was neutralized with 10% NaOH. The oily product was extracted into ethylacetate and from the organic solution evaporated to dryness the title compound was obtained (10 g) as a brown oil.

0094727

Example 17

N-Cyano-N'-/¯4-(pyrazol-3-yl)-phenyl_7 formamidine

A solution of 3-(4-aminophenyl)-pyrazole (15.9 g) and N-cyano ethyl formimidate (9.8 g) in acetone (100 ml) was stirred overnight at room temperature. The solid which crystallized out was filtered and dried to give 12.5 g of the title compound, M.p. 223-224°C.

By utilizing the above procedure the following compounds were prepared:

- N-Cyano-N'-/¯3-(pyrazol-3-yl)-phenyl_7 formamidine
  M.p. 194-197°C

- N-Cyano-N'-/¯4-(imidazo-4-yl)-2-methyl-phenyl_7 formamidine
  M.p. 200-202°C

- N-Cyano-N'-/¯4-(imidazol-4-yl)-2-bromo-phenyl_7 formamidine
  M.p. 183-185°C

- N-Cyano-N'-methyl-N'-/¯4-(imidazol-4-yl)-phenyl_7 formamidine
  M.p. 238-239°C

- N-Cyano-N'-/¯4-(1-methyl-imidazol-4-yl)-phenyl_7 formamidine
  M.p. 233-5°C

- N-Cyano-N'-/¯4-(1-methyl-imidazol-4-yl)-phenyl_7 formamidine
  M.p. 235-7°C

0094727

- N-Cyano-N'-/¯3-(5-methyl-1,2,4-oxadiazol-3-yl)-phenyl_7 formamidine M.p. 179-180°C

- N-Cyano-N'-/¯4-(5-methylamino-1,2,4-oxadiazol-3-yl)-phenyl_7 formamidine M.p. 238-239°C

- N-Cyano-N'-/¯3-(5-methylamino-1,2,4-oxadiazol-3-yl)-phenyl_7 formamidine M.p. 194-195°C

- N-Cyano-N'-/¯4-(2-methyl-oxazol-5-yl)-phenyl_7 formamidine M.p. 196-197°C

- N-Cyano-N'-{3-/¯5-(morpholinomethyl)-furan-2-yl_7-phenyl} formamidine M.p. 162-164°C

- N-Cyano-N'-{4-/¯5-(N,N-diethylaminomethyl)-furan-2-yl_7-phenyl} formamidine M.p. 98-100°C

- N-Cyano-N'-{4-/¯5-(N-methylaminomethyl)-furan-2-yl_7-phenyl} formamidine Yellow oil

- N-Cyano-N'-{4-/¯5-(Pyrrolidinomethyl)-furan-2-yl_7-phenyl} formamidine Brown oil

- N-Cyano-N'-{4-/¯5-(N,N-dimethylaminomethyl)-furan-2-yl_7-phenyl} formamidine Yellow oil

- N-Cyano-N'-{4-/¯5-(N,N-dimethylaminomethyl)-1,2,4-oxadiazol--3-yl_7-phenyl} formamidine M.p. 154-155°C

0094727

- N-Cyano-N'-/‾4-(imidazol-4-yl)-2-chloro-phenyl_7 formamidine

  M.p. 170-171°C

- N-Cyano-N'-/‾4-(imidazol-4-yl)-2,6-dichlorophenyl_7 formamidine

  M.p. 158-160°C

- N-Cyano-N'-/‾4-(imidazol-4-yl)-2-chloro-6-bromophenyl_7 formamidine

  M.p. 178-179°C

Example 18

N-Cyano-N'-/‾4-(imidazol-2-yl)-phenyl_7 formamidine

A mixture of 2-(4-aminophenyl)-1-H-imidazole (3.1 g), cyanamide (0.8 g) and triethylorthoformate (3.6 g) was heated at 150°C for a few minutes. The crystallized product was filtered washed with ethanol and dried to give 2.5 g of the title compound, M.p. 228-229°C.

Similarly were prepared:

| | |
|---|---|
| N-Cyano-N'-/‾3-(imidazol-2-yl)-phenyl_7 formamidine | M.p. 202-203°C |
| N-Cyano-N'-/‾4-(pyridyn-3-yl)-phenyl_7 formamidine | M.p. 227-8°C |
| N-Cyano-N'-/‾3-(pyridyn-2-yl)-phenyl_7 formamidine | M.p. 150-151°C |
| N-Cyano-N'-/‾4-(5-methyl-1,2,4-oxadiazol-3-yl)--phenyl_7 formamidine | M.p. 216-218°C |

Example 19

N-Cyano-N'-/‾4-(imidazo-4-yl)-2-methoxyphenyl_/ formamidine

A solution of 4-(3-methoxy-4-aminophenyl)-1-H-imidazole (9.85 g) and N-cyano ethyl formimidate (5.1 g) in ethanol (100 ml) was stirred overnight at room temperature. The solid, which crystallized out, was filtered and dried to give 12.5 g of the title compound, M.p. 112-116°C.

Similarly were prepared the following compounds:

- N-Cyano-N'-/‾4-(5-bromo-imidazol-4-yl)-phenyl_/ formamidine
  M.p. 203-204°C

- N-Cyano-N'-/‾4-(pyridyn-2-yl)-phenyl_/ formamidine
  M.p. 150-153°C

- N-Cyano-N'-/‾4-(pyrimidon-5-yl)-phenyl_/ formamidine
  M.p. 265-268°C

- N-Cyano-N'-/‾3-(2-methyl-oxazol-5-yl)-phenyl_/ formamidine
  M.p. 218-220°C

- N-Cyano-N'-{3-/‾5-(N,N-dimethylaminomethyl)-furan-2-yl_/-phenyl} formamidine M.p. 186-187°C

- N-Cyano-N'-{4-/‾5-(N,N-dimethylaminomethyl)-thiophen-2-yl_/-phenyl} formamidine M.p. 180-182°C

- N-Cyano-N'-/¯4-(pyrimidin-4-yl)-phenyl_7 formamidine

  M.p. 243-245°C

- N-Cyano-N'-/¯4-(1,2,4-triazol-3-yl)-phenyl_7 formamidine

  M.p. 247-250°C (dec.)

- N-Cyano-N'-/¯4-(2-dimethylaminomethyl-4-thiazolyl)-phenyl_7 formamidine    M.p. 183-185°C

- N-Cyano-N'-/¯3-(2-dimethylaminomethyl-4-thiazolyl)-phenyl_7 formamidine    M.p. 138-140°C

- N-Cyano-N'-{4-/¯2-(1-piperidinylmethyl)-4-thiazolyl_7-phenyl} formamidine    M.p. 183-185°C

- N-Cyano-N'-/¯3-(2-amino-4-thiazolyl)-phenyl_7 formamidine

  M.p. 152-155°C

- N-Cyano-N'-/¯3-(2-methylamino-4-thiazolyl)-phenyl_7 formamidine

  M.p. 181-183°C

- N-Cyano-N'-/¯4-(2-methylamino-4-thiazolyl)-phenyl_7 formamidine

  M.p. 192-196°C (dec.)

- N-Cyano-N'-/¯3-(2-methyl-4-thiazolyl)-phenyl_7 formamidine

  M.p. 202-205°C

- N-Cyano-N'-/¯4-(2-methyl-4-thiazolyl)-phenyl_7 formamidine

  M.p. 210-212°C

- N-Cyano-N'-/¯4-(4-thiazolyl)-phenyl_7 formamidine

  M.p. 218-220°C

Example 20

N-Cyano-N'-/¯4-(5-nitro-imidazol-4-yl)-phenyl_7 formamidine

To a solution of 5-nitro-4-(4-aminophenyl)-1-H-imidazole, sodium salt (17.88 g) in 95% ethanol (70 ml) was added dropwise N-cyano-ethylformimidate (10.2 g). After 30 minutes stirring the separated solid was filtered, washed with 5% acetic acid and ethanol to give 9 g of the title compound, M.p. > 270°C.

In the same manner was prepared:

- N-Cyano-N'-/¯4-(2-mercapto-imidazol-4-yl)-phenyl_7 formamidine M.p. 270°C (dec.).

Example 21

N-(2-Methoxy ethyl)-N'-/‾4-(imidazol-4-yl)-phenyl_/ formamidine (Compound 1)

2-Methoxy ethylamine (5 ml) was added in one portion to a suspension of N-cyano-N'-/‾4-(imidazol-4-yl)-phenyl_/ formamidine (1.8 g) in water (2 ml).When the exothermic reaction subsided a solution was obtained from which the title compound quickly crystallized out. The compound was filtered and purified through its maleate salt in acetone.g 2.8, M.p. 146-149°C (dec.).

Analysis

| | | | | | | | |
|---|---|---|---|---|---|---|---|
| $C_{21}H_{24}N_4O_9$ | Found % | C | 52.60 | H | 5.06 | N | 11.56 |
| | Calc. % | C | 52.94 | H | 5.08 | N | 11.76 |

Substitution of the suitable N-cyano formamidine derivatives and amino compounds for those ones of Example 21 and utilizing the procedure here described led to the production of the following formamidines.

N-(2-Hydroxy propyl)-N'-/‾4-(imidazol-4-yl)-phenyl_/ formamidine (Compound 2)

Maleate salt (ethanol) M.p. 140-144°C (dec.)

Analysis

| | | | | | | | |
|---|---|---|---|---|---|---|---|
| $C_{21}H_{24}N_4O_9$ | Found % | C | 53.04 | H | 5.11 | N | 11.48 |
| | Calc. % | C | 52.94 | H | 5.08 | N | 11.76 |

N-(2-Methyl thio ethyl)-N'-/¯4-(imidazol-4-yl)-phenyl_7 formamidine

(Compound 3)

Maleate salt (ethanol 95%) M.p. 145-146°C (dec.)

Analysis

| | | | | |
|---|---|---|---|---|
| $C_{21}H_{24}N_4O_8S$ | Found % | C 51.15 | H 5.02 | N 11.36 |
| | Calc. % | C 51.21 | H 4.91 | N 11.38 |

N-(2.2-Dimethoxy ethyl)-N'-/¯4-(imidazol-4-yl)-phenyl_7 formamidine

(Compound 4)

Maleate salt (ethanol) M.p. 143°C (dec.)

Analysis

| | | | | |
|---|---|---|---|---|
| $C_{22}H_{26}N_4O_{10}$ | Found % | C 52.01 | H 5.38 | N 10.96 |
| | Calc. % | C 52.17 | H 5.18 | N 11.06 |

N-(3-Methoxy propyl)-N'-/¯4-imidazol-4-yl)-phenyl_7 formamidine

(Compound 5)

Maleate salt (ethanol) M.p. 145-146°C (dec.)

Analysis

| | | | | |
|---|---|---|---|---|
| $C_{22}H_{26}N_4O_9$ | Found % | C 53.61 | H 5.16 | N 11.22 |
| | Calc. % | C 53.87 | H 5.34 | N 11.42 |

N-(2-Ethyl thio ethyl)-N'-/‾4-(imidazol-4-yl)-phenyl_7 formamidine

(Compound 6)

Hydrochloride salt (ethanol) M.p. 126-129°C (dec.)

Analysis

| | | | | |
|---|---|---|---|---|
| $C_{14}H_{20}Cl_2N_4S$ | Found % | C 48.00 | H 6.01 | N 15.98 |
| | Calc. % | C 48.41 | H 5.80 | N 16.13 |

N-(3-Hydroxy propyl)-N'-/‾4-(imidazol-4-yl)-phenyl_7 formamidine

(Compound 7)

Maleate salt (ethanol) M.p. 138-139°C (dec.)

Analysis

| | | | | |
|---|---|---|---|---|
| $C_{21}H_{24}N_4O_9$ | Found % | C 53.19 | H 4.99 | N 11.52 |
| | Calc. % | C 52.94 | H 5.08 | N 11.76 |

N-(1-Hydroxy methyl propyl)-N'-/‾4-(imidazol-4-yl)-phenyl_7 formamidine

(Compound 8)

Maleate salt (ethanol) M.p. 172-173°C (dec.)

Analysis

| | | | | |
|---|---|---|---|---|
| $C_{22}H_{26}N_4O_9$ | Found % | C 54.02 | H 5.30 | N 11.51 |
| | Calc. % | C 53.87 | H 5.34 | N 11.42 |

N-/‾(2.2-Dimethyl-1.3-dioxolan-4-yl)-methyl_7-N'-/‾4-(imidazol-4--yl)-phenyl_7 formamidine (Compound 9)

Maleate salt (ethanol) M.p. 179-180°C (dec.)

Analysis

| | | | | |
|---|---|---|---|---|
| $C_{24}H_{28}N_4O_{10}$ | Found % | C 54.16 | H 5.25 | N 10.67 |
| | Calc. % | C 54.12 | H 5.30 | N 10.52 |

N-(3-N,N-Dimethyl amino propyl)-N'-/‾4-(imidazol-4-yl)-phenyl_7 formamidine (Compound 10)

Maleate salt (ethanol) M.p. 138-139°C (dec.)

Analysis

| | | | | |
|---|---|---|---|---|
| $C_{27}H_{33}N_5O_{12}$ | Found % | C 51.91 | H 5.33 | N 11.09 |
| | Calc. % | C 52.34 | H 5.37 | N 11.30 |

N-(2-Cyano ethyl)-N'-/‾4-(imidazol-4-yl)-phenyl_7 formamidine (Compound 11)

Maleate salt (ethanol) M.p. 140-141°C (dec.)

Analysis

| | | | | |
|---|---|---|---|---|
| $C_{21}H_{21}N_5O_8$ | Found % | C 53.03 | H 4.50 | N 14.74 |
| | Calc. % | C 53.50 | H 4.49 | N 14.86 |

N-n-Butyl-N'-/‾4-(imidazo-4-yl)-2-methyl-phenyl_/ formamidine

(Compound 12)

Fumarate salt (ethanol) M.p. 168-170°C (dec.)

Analysis

| | | | | | | | |
|---|---|---|---|---|---|---|---|
| $C_{23}H_{28}N_4O_8$ | Found % | C | 56.70 | H | 6.00 | N | 11.49 |
| | Calc. % | C | 56.55 | H | 5.78 | N | 11.47 |

N-Allyl-N'-/‾4-(imidazol-4-yl)-2-methyl-phenyl_/ formamidine

(Compound 13)

Maleate salt (acetone) M.p. 148-150°C (dec.)

Analysis

| | | | | | | | |
|---|---|---|---|---|---|---|---|
| $C_{22}H_{24}N_4O_8$ | Found % | C | 55.63 | H | 5.02 | N | 11.65 |
| | Calc. % | C | 55.93 | H | 5.12 | N | 11.86 |

N-Propyl-N'-/‾2-methoxy-4-(imidazol-4-yl)-phenyl_/ formamidine

(Compound 14)

Maleate salt (acetone) M.p. 165-167°C (dec.)

Analysis

| | | | | | | | |
|---|---|---|---|---|---|---|---|
| $C_{22}H_{26}N_4O_9$ | Found % | C | 53.49 | H | 5.38 | N | 11.33 |
| | Calc. % | C | 53.87 | H | 5.34 | N | 11.42 |

N-Ethyl-N'-/2-methoxy-4-(imidazol-4-yl)-phenyl/ formamidine

(Compound 15)

Maleate salt (acetone) M.p. 162°C (dec.)

Analysis

| | | | | | | | | |
|---|---|---|---|---|---|---|---|---|
| $C_{21}H_{24}N_4O_9$ | Found % | C | 52.65 | H | 4.94 | N | 11.31 |
| | Calc. % | C | 52.94 | H | 5.08 | N | 11.76 |

N-Isopropyl-N'-/4-(5-nitro-imidazol-4-yl)-phenyl/ formamidine

(Compound 16)

Hydrochloride salt (acetone) M.p. 242-3°C (dec.)

Analysis

| | | | | | | | |
|---|---|---|---|---|---|---|---|
| $C_{13}H_{16}ClN_5O_2$ | Found % | C | 50.12 | H | 5.39 | N | 22.53 |
| | Calc. % | C | 50.40 | H | 5.21 | N | 22.61 |

N-Isopropyl-N'-/4-(5-bromo-imidazol-4-yl)-phenyl/ formamidine

(Compound 17)

Fumarate salt (acetone) M.p. 196°C (dec.)

Analysis

| | | | | | | | |
|---|---|---|---|---|---|---|---|
| $C_{21}H_{23}BrN_4O_8$ | Found % | C | 46.82 | H | 4.25 | N | 10.51 |
| | Calc. % | C | 46.76 | H | 4.29 | N | 10.39 |

N-Methyl-N-isopropyl-N'-/¯4-(imidazol-4-yl)-phenyl_7 formamidine

(Compound 18)

Hydrochloride salt (ethanol) M.p. 254-255°C (dec.)

Analysis

| | | | | |
|---|---|---|---|---|
| $C_{14}H_{20}Cl_2N_4$ | Found % | C 53.20 | H 6.44 | N 17.55 |
| | Calc. % | C 53.33 | H 6.39 | N 17.77 |

N-Isopropyl-N'-methyl-N'-/¯4-(imidazol-4-yl)-phenyl_7 formamidine

(Compound 19)

Maleate salt (ethanol) M.p. 157-158°C (dec.)

Analysis

| | | | | |
|---|---|---|---|---|
| $C_{22}H_{26}N_4O_8$ | Found % | C 55.23 | H 5.49 | N 11.62 |
| | Calc. % | C 55.69 | H 5.52 | N 11.81 |

N-n-Propyl-N'-methyl-N'-/¯4-(imidazol-4-yl)-phenyl_7 formamidine

(Compound 20)

Maleate salt (acetone) M.p. 139-140°C (dec.)

Analysis

| | | | | |
|---|---|---|---|---|
| $C_{22}H_{26}N_4O_8$ | Found % | C 55.47 | H 5.39 | N 11.75 |
| | Calc. % | C 55.69 | H 5.52 | N 11.81 |

N-Propyl-N'-/¯4-/¯5-(N,N-dimethylamino methyl)-thiophen-2-yl_7--phenyl_7 formamidine (Compound 21)

Maleate salt (acetone) M.p. 111-115°C (dec.)

Analysis

| | | | | |
|---|---|---|---|---|
| $C_{25}H_{31}N_3O_8S$ | Found % | C 56.58 | H 5.91 | N 7.97 |
| | Calc. % | C 56.27 | H 5.86 | N 7.87 |

N-sec-Butyl-N'-/¯4-/¯5-(N,N-dimethylaminomethyl)-thiophen-2-yl_7--phenyl_7 formamidine (Compound 22)

Fumarate salt (acetone) M.p. 195-197°C (dec.)

Analysis

| | | | | |
|---|---|---|---|---|
| $C_{30}H_{37}N_3O_{12}S$ | Found % | C 58.63 | H 6.49 | N 8.70 |
| | Calc. % | C 58.87 | H 6.38 | N 8.58 |

N-Isopropyl-N'-/¯4-(pyrazol-3-yl)-phenyl_7 formamidine (Compound 23)

Hydrochloride salt (acetone) M.p. 202-203°C (dec.)

Analysis

| | | | | |
|---|---|---|---|---|
| $C_{13}H_{18}Cl_2N_4$ | Found % | C 51.57 | H 5.98 | N 18.28 |
| | Calc.% | C 51.83 | H 6.02 | N 18.60 |

N-Allyl-N'-/¯4-(pyrazol-3-yl)-phenyl_7 formamidine

(Compound 24)

Hydrochloride salt (acetone) M.p. 212-213°C (dec.)

Analysis

| | | | | |
|---|---|---|---|---|
| $C_{13}H_{16}Cl_2N_4$ | Found % | C 52.42 | H 5.48 | N 18.78 |
| | Calc. % | C 52.18 | H 5.39 | N 18.73 |

N-sec-Butyl-N'-/¯4-(pyrazol-3-yl)-phenyl_7 formamidine

(Compound 25)

Hydrochloride salt (acetone) M.p. 209-210°C (dec.)

Analysis

| | | | | |
|---|---|---|---|---|
| $C_{14}H_{20}Cl_2N_4$ | Found % | C 53.23 | H 6.60 | N 17.66 |
| | Calc. % | C 53.34 | H 6.39 | N 17.77 |

N-Propyl-N'-/¯4-(pyrazol-3-yl)-phenyl_7 formamidine

(Compound 26)

Hydrochloride salt (ethanol) M.p. 199-201°C (dec.)

Analysis

| | | | | |
|---|---|---|---|---|
| $C_{13}H_{18}Cl_2N_4$ | Found % | C 51.23 | H 6.09 | N 18.54 |
| | Calc. % | C 51.18 | H 6.02 | N 18.60 |

N-(2-Methyl thio ethyl)-N'-/¯4-(pyrazol-3-yl)-phenyl_7 formamidine

(Compound 27)

Nitrate salt (ethanol) M.p. 141°C (dec.)

Analysis

| | | | | |
|---|---|---|---|---|
| $C_{13}H_{17}N_5O_3S$ | Found % | C 48.11 | H 5.32 | N 21.73 |
| | Calc. % | C 48.28 | H 5.29 | N 21.66 |

N-Methyl-N'-/¯4-(imidazol-2-yl)-phenyl_7 formamidine

(Compound 28)

Fumarate salt (ethanol) M.p. 195-198°C (dec.)

Analysis

| | | | | |
|---|---|---|---|---|
| $C_{34}H_{36}N_8O_{12}$ | Found % | C 54.21 | H 4.78 | N 15.09 |
| | Calc. % | C 54.54 | H 4.85 | N 14.97 |

N-Isopropyl-N'-/¯4-(imidazol-2-yl)-phenyl_7 formamidine

(Compound 29)

Fumarate salt (ethanol) M.p. 135-140°C (dec.)

Analysis

| | | | | |
|---|---|---|---|---|
| $C_{21}H_{24}N_4O_8$ | Found % | C 55.78 | H 5.38 | N 12.25 |
| | Calc. % | C 54.77 | H 5.25 | N 12.17 |

N-Isopropyl-N'-[4-(2-mercapto-imidazol-4-yl)-phenyl] formamidine

(Compound 30)

Maleate salt (acetone) M.p. 202°C (dec.)

Analysis

| | | | | |
|---|---|---|---|---|
| $C_{17}H_{20}H_4O_4S$ | Found % | C 54.38 | H 5.33 | N 14.70 |
| | Calc. % | C 54.24 | H 5.35 | N 14.88 |

N-Isopropyl-N'-[4-(1-methyl-imidazol-4-yl)-phenyl] formamidine

(Compound 31)

Hydrochloride salt (ethanol-acetone) M.p. 228-230°C (dec.)

Analysis

| | | | | |
|---|---|---|---|---|
| $C_{14}H_{20}Cl_2N_4$ | Found % | C 52.98 | H 6.45 | N 17.58 |
| | Calc. % | C 53.33 | H 6.39 | N 17.77 |

N-Isopropyl-N'-[4-(1-methyl-imidazol-5-yl)-phenyl] formamidine

(Compound 32)

Hydrochloride salt (ethanol-acetone) M.p. 180-182°C (dec.)

Analysis

| | | | | |
|---|---|---|---|---|
| $C_{14}H_{20}Cl_2N_4$ | Found % | C 53.41 | H 6.42 | N 17.60 |
| | Calc. % | C 53.33 | H 6.39 | N 17.77 |

N-Isopropyl-N'-/‾3-(5-methyl-1.2.4-oxadiazol-3-yl)-phenyl_7 formamidine

(Compound 33)

Hydrochloride salt (acetone) M.p. 218-220°C (dec.)

Analysis

| | | | | | | | |
|---|---|---|---|---|---|---|---|
| $C_{13}H_{17}ClN_4O$ | Found % | C | 55.24 | H | 6.15 | N | 19.92 |
| | Calc. % | C | 55.61 | H | 6.10 | N | 19.96 |

N-neo Pentyl-N'-/‾4-(pyridimidin-4-yl)-phenyl_7 formamidine

(Compound 34)

Maleate salt (ethanol) M.p. 165-167°C (dec.)

Analysis

| | | | | | | | |
|---|---|---|---|---|---|---|---|
| $C_{20}H_{20}N_4O_4$ | Found % | C | 62.81 | H | 6.40 | N | 14.44 |
| | Calc. | C | 62.48 | H | 6.29 | N | 14.58 |

N-Isopropyl-N'-/‾4-(pyridimidin-4-yl)-phenyl_7 formamidine

(Compound 35)

Fumarate salt (ethanol) M.p. 190-191°C (dec.)

Analysis

| | | | | | | | |
|---|---|---|---|---|---|---|---|
| $C_{40}H_{44}N_8O_{12}$ | Found % | C | 57.51 | H | 5.44 | N | 13.42 |
| | Calc. % | C | 57.96 | H | 5.35 | N | 13.52 |

N-Isopropyl-N'-[4-(2-methyl-oxazol-5-yl)-phenyl] formamidine

(Compound 36)

Nitrate salt (acetone) M.p. 156-157°C (dec.)

Analysis

| | | | | | | | |
|---|---|---|---|---|---|---|---|
| $C_{14}H_{19}N_5O_7$ | Found % | C | 45.45 | H | 5.10 | N | 18.55 |
| | Calc. % | C | 45.53 | H | 5.19 | N | 18.96 |

N-Isopropyl-N'-[4-(1.2.4-triazol-3-yl)-phenyl] formamidine

(Compound 86)

Maleate salt (ethanol) M.p. 156-157°C (dec.)

Analysis

| | | | | | | | |
|---|---|---|---|---|---|---|---|
| $C_{20}H_{23}N_5O_8$ | Found % | C | 51.97 | H | 5.05 | N | 15.40 |
| | Calc. % | C | 52.06 | H | 5.02 | N | 15.18 |

N-Propyl-N'-[4-(1.2.4-triazol-3-yl)-phenyl] formamidine

(Compound 87)

Maleate salt (acetone) M.p. 140-142°C (dec.)

Analysis

| | | | | | | | |
|---|---|---|---|---|---|---|---|
| $C_{20}H_{23}N_5O_8$ | Found % | C | 51.83 | H | 5.15 | N | 15.00 |
| | Calc. % | C | 52.06 | H | 5.02 | N | 15.18 |

N-sec-Butyl-N'-/‾4-(1.2.4-triazol-3-yl)-phenyl_7 formamidine

(Compound 88)

Maleate salt (acetone) M.p. 152-154°C (dec.)

Analysis

| | | | | |
|---|---|---|---|---|
| $C_{21}H_{25}N_5O_8$ | Found % | C 52.77 | H 5.32 | N 14.64 |
| | Calc. % | C 53.05 | H 5.30 | N 14.73 |

N-Allyl-N'-/‾4-(1.2.4-triazol-3-yl)-phenyl_7 formamidine

(Compound 90)

Maleate salt (acetone) M.p. 146-148°C (dec.)

Analysis

| | | | | |
|---|---|---|---|---|
| $C_{16}H_{17}N_5O_4$ | Found % | C 55.64 | H 5.04 | N 20.46 |
| | Calc. % | C 55.97 | H 4.99 | N 20.40 |

N-(2-Methoxyethyl)-N'-/‾4-(1.2.4-triazol-3-yl)-phenyl_7 formamidine

(Compound 91)

Fumarate salt (acetone) M.p. 182-184°C (dec.)

Analysis

| | | | | |
|---|---|---|---|---|
| $C_{16}H_{19}N_5O_5$ | Found % | C 53.01 | H 5.35 | N 19.53 |
| | Calc. % | C 53.18 | H 5.30 | N 19.38 |

N-Propyl-N'-/‾4-(2-dimethylaminomethyl-4-thiazolyl)-phenyl_7 formamidine (Compound 93)

M.p. 107-108°C (dec.)

Analysis

| | | | | |
|---|---|---|---|---|
| $C_{16}H_{22}N_4S$ | Found % | C 63.77 | H 7.25 | N 18.56 |
| | Calc. % | C 63.54 | H 7.33 | N 18.52 |

N-Isopropyl-N'-/‾4-(2-dimethylaminomethyl-4-thiazolyl)-phenyl_7 formamidine (Compound 94)

M.p. 139-143°C (dec.)

Analysis

| | | | | |
|---|---|---|---|---|
| $C_{16}H_{22}N_4S$ | Found % | C 63.71 | H 7.39 | N 18.82 |
| | Calc. % | C 63.54 | H 7.33 | N 18.52 |

N-Allyl-N'-/‾4-(2-dimethylaminomethyl-4-thiazolyl)-phenyl_7 formamidine (Compound 95)

M.p. 112-113°C (dec.)

Analysis

| | | | | |
|---|---|---|---|---|
| $C_{16}H_{20}N_4S$ | Found % | C 63.59 | H 6.67 | N 18.67 |
| | Calc. % | C 63.97 | H 6.71 | N 18.65 |

N-sec-Butyl-N'-/¯4-(2-dimethylaminomethyl-4-thiazolyl)-phenyl_7 formamidine (Compound 96)

M.p. 97-98°C (dec.)

Analysis

| | | | | |
|---|---|---|---|---|
| $C_{17}H_{24}N_4S$ | Found % | C 64.68 | H 7.60 | N 17.82 |
| | Calc. % | C 64.52 | H 7.64 | N 17.70 |

N-Neopentyl-N'-/¯4-(2-dimethylaminomethyl-4-thiazolyl)-phenyl_7 formamidine (Compound 97)

M.p. 115-118°C (dec.)

Analysis

| | | | | |
|---|---|---|---|---|
| $C_{18}H_{26}N_4S$ | Found % | C 65.34 | H 7.91 | N 16.98 |
| | Calc. % | C 65.42 | H 7.93 | N 16.95 |

N-Hexyl-N'-/¯4-(2-dimethylaminomethyl)-4-thiazolyl)-phenyl_7 formamidine (Compound 98)

M.p. 64-65°C (dec.)

Analysis

| | | | | |
|---|---|---|---|---|
| $C_{19}H_{28}N_4S$ | Found % | C 65.81 | H 8.26 | N 16.19 |
| | Calc. % | C 66.24 | H 8.19 | N 16.26 |

N-Octyl-N'-/‾4-(2-dimethylaminomethyl-4-thiazolyl)-phenyl_7 formamidine (Compound 99)

M.p. 83-85°C (dec.)

Analysis

| | | | | |
|---|---|---|---|---|
| $C_{21}H_{32}N_4S$ | Found % | C 67.65 | H 8.82 | N 15.12 |
| | Calc. % | C 67.69 | H 8.66 | N 15.04 |

N-Isopropyl-N'-{4-/‾2-(1-piperidinyl-methyl)-4-thiazolyl_7-phenyl} formamidine (Compound 101)

Maleate salt (acetone) M.p. 157-160°C (dec.)

Analysis

| | | | | |
|---|---|---|---|---|
| $C_{27}H_{34}N_4O_8S$ | Found % | C 56.69 | H 6.03 | N 9.79 |
| | Calc. % | C 56.43 | H 5.96 | N 9.75 |

N-Neopentyl-N'-{4-/‾2-(1-piperidinyl-methyl)-4-thiazolyl_7-phenyl} formamidine (Compound 102)

Maleate salt (acetone) M.p. 180-181°C (dec.)

Analysis

| | | | | |
|---|---|---|---|---|
| $C_{29}H_{38}N_4O_8S$ | Found % | C 58.03 | H 6.42 | N 9.40 |
| | Calc. % | C 57.59 | H 6.35 | N 9.30 |

N-Isopropyl-N'-[3-(2-methyl-4-thiazolyl)-phenyl] formamidine

(Compound 107)

M.p. 110-112°C (dec.)

Analysis

| | | | | |
|---|---|---|---|---|
| $C_{14}H_{17}N_3S$ | Found % | C 63.56 | H 6.85 | N 16.30 |
| | Calc. % | C 63.83 | H 6.61 | N 16.20 |

N-Ethyl-N'-[4-(2-methyl-4-thiazolyl)-phenyl] formamidine

(Compound 108)

M.p. 119-120°C (dec.)

Analysis

| | | | | |
|---|---|---|---|---|
| $C_{13}H_{15}N_3S$ | Found % | C 63.41 | H 6.18 | N 17.01 |
| | Calc. % | C 63.64 | H 6.16 | N 17.13 |

N-Propyl-N'-[4-(2-methyl-4-thiazolyl)-phenyl] formamidine

(Compound 109)

M.p. 112-115°C (dec.)

Analysis

| | | | | |
|---|---|---|---|---|
| $C_{14}H_{17}N_3S$ | Found % | C 64.04 | H 6.72 | N 15.96 |
| | Calc. % | C 63.83 | H 6.61 | N 16.20 |

N-Isopropyl-N'-[4-(2-methyl-4-thiazolyl)-phenyl] formamidine (Compound 110)

M.p. 119-120°C (dec.)

Analysis

| | | | | |
|---|---|---|---|---|
| $C_{14}H_{17}N_3S$ | Found % | C 63.52 | H 6.42 | N 16.20 |
| | Calc. % | C 63.83 | H 6.61 | N 16.20 |

N-Isopropyl-N'-[4-(4-thiazolyl)-phenyl] formamidine (Compound 111)

M.p. 110-113°C (dec.)

Analysis

| | | | | |
|---|---|---|---|---|
| $C_{13}H_{15}N_3S$ | Found % | C 63.24 | H 5.91 | N 17.34 |
| | Calc. % | C 63.66 | H 6.16 | N 17.13 |

N-Propargyl-N'-[4-(4-thiazolyl)-phenyl] formamidine (Compound 112)

M.p. 129-132°C (dec.)

Analysis

| | | | | |
|---|---|---|---|---|
| $C_{13}H_{11}N_3S$ | Found % | C 64.40 | H 4.49 | N 17.42 |
| | Calc. % | C 64.72 | H 4.60 | N 17.42 |

Example 22

N-Methyl-N'-/‾3-/‾ 5-(N,N-dimethylamino methyl)-furan-2-yl_7-phenyl_7 formamidine (Compound 37)

N-Cyano-N'-/‾3-/‾5-(N,N-dimethylamino methyl)-furan-2-yl_7-phenyl_7 formamidine (3 g) was allowed to react with a 35% methylamine solution in water (7 ml). After 5 minutes stirring water (60 ml) was added to the solution and the separated oil was taken up into ethylacetate. This was washed with water, dried and evaporated to dryness to give 3.3 g of the title compound as a thick oil. The base was purified through its fumarate solution ethylacetate, M.p. 75–76°C.

Analysis

| | | | | |
|---|---|---|---|---|
| $C_{23}H_{27}N_3O_9$ | Found % | C 55.69 | H 5.61 | N 8.63 |
| | Calc. % | C 56.43 | H 5.56 | N 8.59 |

Similarly were prepared the following compounds:

N-Isopropyl-N'-/‾3-/‾5-(N,N-dimethylamino methyl)-furan-2-yl_7--phenyl_7 formamidine (Compound 38)

Tartrate salt (acetone) M.p. 90°C (dec.)

Analysis

| | | | | |
|---|---|---|---|---|
| $C_{27}H_{35}N_3O_{13}$ | Found % | C 51.27 | H 6.04 | N 7.24 |
| | Calc. % | C 51.27 | H 6.03 | N 7.17 |

N-sec-Butyl-N'-/‾3-/‾5-(morpholino methyl)-furan-2-yl_7-phenyl_7 formamidine (Compound 39)

Maleate salt (ethanol) M.p. 158-160°C (dec.)

Analysis

| | | | | |
|---|---|---|---|---|
| $C_{28}H_{35}N_3O_{10}$ | Found % | C 58.76 | H 6.33 | N 7.27 |
| | Calc. % | C 58.63 | H 6.15 | N 7.33 |

N-Cyclohexyl-N'-/‾3-/‾5-(N,N-dimethylamino methyl)-furan-2-yl_7-phenyl_7 formamidine (Compound 40)

Tartrate salt (acetone) M.p. 105-106°C

Analysis

| | | | | |
|---|---|---|---|---|
| $C_{28}H_{39}N_3O_{13}$ | Found % | C 53.52 | H 6.16 | N 6.93 |
| | Calc. % | C 53.75 | H 6.28 | N 6.72 |

N-Methyl-N'-/‾4-/‾5-(N,N-dimethylamino methyl)-furan-2-yl_7--phenyl_7 formamidine (Compound 41)

Maleate salt (acetone) M.p. 142-4°C (dec.)

Analysis

| | | | | |
|---|---|---|---|---|
| $C_{23}H_{27}N_3O_9$ | Found % | C 56.07 | H 5.41 | N 8.50 |
| | Calc. % | C 56.43 | H 5.56 | N 8.58 |

N-Isopropyl-N'-/ 4-/ 5-(N,N-dimethylamino methyl)-furan-2-yl_7-

-phenyl_7 formamidine (Compound 42)

Maleate salt (acetone) M.p. 142-5°C (dec.)

Analysis

| | | | | |
|---|---|---|---|---|
| $C_{25}H_{31}N_3O_9$ | Found % | C 57.93 | H 5.87 | N 8.17 |
| | Calc. % | C 58.02 | H 6.04 | N 8.12 |

N-Allyl-N'-/ 4-/ 5-(N,N-dimethylamino methyl)-furan-2-yl_7-phenyl_7

formamidine (Compound 43)

Maleate salt (acetone) M.p. 153-5°C (dec.)

Analysis

| | | | | |
|---|---|---|---|---|
| $C_{25}H_{29}N_3O_9$ | Found % | C 57.96 | H 5.89 | N 8.04 |
| | Calc. % | C 58.24 | H 5.67 | N 8.15 |

N-n-Butyl-N'-/ 4-/ 5-(N,N-dimethylamino methyl)-furan-2-yl_7- phenyl_7

formamidine (Compound 44)

Maleate salt (acetone) M.p. 129-130°C

Analysis

| | | | | |
|---|---|---|---|---|
| $C_{26}H_{33}N_3O_9$ | Found % | C 58.45 | H 6.34 | N 7.76 |
| | Calc. % | C 58.75 | H 6.26 | N 7.91 |

N-sec-Butyl-N'-/‾4-/‾5-(N-methylamino methyl)-furan-2-yl_7-phenyl_7 formamidine (Compound 45)

Tartrate salt (ethylacetate-acetone) M.p. 60°C (dec.)

Analysis

| | | | | |
|---|---|---|---|---|
| $C_{21}H_{29}N_3O_7$ | Found % | C 57.58 | H 6.72 | N 9.44 |
| | Calc. % | C 57.92 | H 6.71 | N 9.65 |

N-Ethyl-N'-/‾4-/‾5-(pyrrolidino methyl)-furan-2-yl_7-phenyl_7 formamidine (Compound 46)

Tartrate salt (ethanol-acetone) M.p. 105-108°C (dec.)

Analysis

| | | | | |
|---|---|---|---|---|
| $C_{48}H_{64}N_6O_{20}$ | Found % | C 55.43 | H 6.16 | N 8.27 |
| | Calc. % | C 55.16 | H 6.17 | N 8.04 |

N-n-Octyl-N'-/‾4-/‾5-(N,N-diethylamino methyl)-furan-2-yl_7-phenyl_7 formamidine (Compound 47)

Tartrate salt (ethylacetate) M.p. 68-70°C (dec.)

Analysis

| | | | | |
|---|---|---|---|---|
| $C_{32}H_{49}N_3O_{13}$ | Found % | C 55.95 | H 7.07 | N 5.99 |
| | Calc. % | C 56.21 | H 7.22 | N 6.15 |

N-neo-Pentyl-N'-[4-[5-(N,N-diethylamino methyl)-furan-2-yl]-phenyl] formamidine (Compound 48)

Tartrate salt (ethylacetato-acetone) M.p. 106-110°C (dec.)

Analysis

| | | | | |
|---|---|---|---|---|
| $C_{29}H_{43}N_3O_{13}$ | Found % | C 54.16 | H 6.56 | N 6.74 |
| | Calc. % | C 54.28 | H 6.75 | N 6.56 |

N-Ethyl-N'-[3-(pyrazol-3-yl)-phenyl] formamidine (Compound 49)

Hydrobromide salt (acetone) M.p. 80-81°C (dec.)

Analysis

| | | | | |
|---|---|---|---|---|
| $C_{12}H_{15}BrN_4$ | Found % | C 48.95 | H 5.23 | N 18.57 |
| | Calc. % | C 48.82 | H 5.12 | N 18.98 |

N-Propyl-N'-[3-(pyrazol-3-yl)-phenyl] formamidine (Compound 50)

Hydrochloride salt (acetone) M.p. 160-162°C (dec.)

Analysis

| | | | | |
|---|---|---|---|---|
| $C_{13}H_{17}ClN_4$ | Found % | C 58.48 | H 6.34 | N 20.98 |
| | Calc. % | C 58.97 | H 6.47 | N 21.16 |

N-n-Hexyl-N'-/¯4-(pyridin-2-yl)-phenyl_7 formamidine

(Compound 54)

Maleate salt (ethylacetate) M.p. 138-140°C (dec.)

Analysis

| | | | | |
|---|---|---|---|---|
| $C_{22}H_{27}N_3O_4$ | Found % | C 66.82 | H 6.87 | N 10.53 |
| | Calc. % | C 66.48 | H 6.85 | N 10.57 |

N-Allyl-N'-/¯4-(pyridin-2-yl)-phenyl_7 formamidine

(Compound 55)

Maleate salt (ethylacetate) M.p. 120°C (dec.)

Analysis

| | | | | |
|---|---|---|---|---|
| $C_{19}H_{19}N_3O_4$ | Found % | C 64.73 | H 5.78 | N 11.29 |
| | Calc. % | C 64.58 | H 5.42 | N 11.89 |

N-sec-Butyl-N'-/¯4-(pyridin-2-yl)-phenyl_7 formamidine

(Compound 56)

Maleate salt (ethylacetate) M.p. 126-127°C (dec.)

Analysis

| | | | | |
|---|---|---|---|---|
| $C_{24}H_{27}N_3O_8$ | Found % | C 59.71 | H 5.44 | N 8.75 |
| | Calc. % | C 59.37 | H 5.61 | N 8.65 |

N-Cyclopropyl-N'-/¯4-(pyridin-2-yl)-phenyl_7 formamidine

(Compound 57)

Maleate salt (ethylacetate) M.p. 130-134°C (dec.)

Analysis

| | | | | |
|---|---|---|---|---|
| $C_{23}H_{23}N_3O_8$ | Found % | C 58.80 | H 4.98 | N 9.09 |
| | Calc. % | C 58.84 | H 5.94 | N 8.95 |

N-Isopropyl-N'-/¯3-(pyridin-2-yl)-phenyl_7 formamidine

(Compound 58)

Tartrate salt (ethylacetate) M.p. 170°C (dec.)

Analysis

| | | | | |
|---|---|---|---|---|
| $C_{19}H_{23}N_3O_6$ | Found % | C 58.92 | H 6.02 | N 10.64 |
| | Calc. % | C 58.60 | H 5.95 | N 10.79 |

N-Allyl-N'-/¯3-(pyridin-2-yl)-phenyl_7 formamidine

(Compound 59)

Tartrate salt (ethylacetate) M.p. 150-154°C (dec.)

Analysis

| | | | | |
|---|---|---|---|---|
| $C_{34}H_{36}N_6O_6$ | Found % | C 65.15 | H 5.77 | N 13.23 |
| | Calc. % | C 65.36 | H 5.80 | N 13.45 |

N-sec-Butyl-N'-/¯4-(imidazol-2-yl-phenyl_7 formamidine (Compound 60)

Fumarate salt (acetone) M.p. 125-126°C (dec.)

Analysis

| | | | | |
|---|---|---|---|---|
| $C_{19}H_{22}N_4O_4$ | Found % | C 61.14 | H 6.01 | N 14.92 |
| | Calc. % | C 61.61 | H 5.99 | N 15.13 |

N-Allyl-N'-/¯3-(imidazol-2-yl)-phenyl_7 formamidine (Compound 61)

Maleate salt (ethanol) M.p. 170-171°C (dec.)

Analysis

| | | | | |
|---|---|---|---|---|
| $C_{21}H_{22}N_4O_8$ | Found % | C 55.27 | H 4.70 | N 12.45 |
| | Calc. % | C 55.02 | H 4.84 | N 12.22 |

N-Propyl-N'-/¯3-(imidazol-2-yl)-phenyl_7 formamidine (Compound 62)

Maleate salt (ethanol) M.p. 172-173°C (dec.)

Analysis

| | | | | |
|---|---|---|---|---|
| $C_{21}H_{24}N_4O_8$ | Found % | C 54.87 | H 5.30 | N 12.31 |
| | Calc. % | C 54.78 | H 5.25 | N 12.17 |

N-Isopropyl-N'-/¯4-(5-methyl-1.2.4-oxadiazol-3-yl)-phenyl_7 formamidine (Compound 63)

Maleate salt (ethylacetate) M.p. 154-155°C (dec.)

Analysis

| | | | | | | | |
|---|---|---|---|---|---|---|---|
| $C_{17}H_{20}N_4O_5$ | Found % | C | 56.89 | H | 5.42 | N | 15.24 |
| | Calc. % | C | 56.66 | H | 5.59 | N | 15.55 |

N-Isopropyl-N'-/¯4-(5-methylamino-1.2.4-oxadiazol-3-yl)-phenyl_7 formamidine (Compound 64)

Fumarate salt (acetone) M.p. 184-5°C (dec.)

Analysis

| | | | | | | | |
|---|---|---|---|---|---|---|---|
| $C_{17}H_{21}N_5O_5$ | Found % | C | 54.04 | H | 5.58 | N | 18.81 |
| | Calc. % | C | 54.39 | H | 5.64 | N | 18.66 |

N-Isopropyl-N'-/¯3-(5-methylamino-1.2.4-oxadiazol-3-yl)-phenyl_7 formamidine (Compound 65)

Fumarate salt (acetone) M.p. 188-9°C (dec.)

Analysis

| | | | | | | | |
|---|---|---|---|---|---|---|---|
| $C_{38}H_{46}N_{10}O_{14}$ | Found % | C | 52.44 | H | 5.46 | N | 16.39 |
| | Calc. % | C | 52.65 | H | 5.35 | N | 16.16 |

N-n-Hexyl-N'-/¯4-(5-N,N-dimethylamino methyl-1.2.4-oxadiazol-3-yl)-phenyl_7 formamidine (Compound 66)

Maleate salt (ethylacetate) M.p. 135-136°C (dec.)

Analysis

| | | | | |
|---|---|---|---|---|
| $C_{26}H_{35}N_5O_9$ | Found % | C 55.10 | H 6.33 | N 12.22 |
| | Calc. % | C 55.60 | H 6.28 | N 12.47 |

N-Isopropyl-N'-/¯4-(5-N,N-dimethylamino methyl-1.2.4-oxadiazol-3-yl)-phenyl_7 formamidine (Compound 67)

Maleate salt (acetone) M.p. 75-76°C (dec.)

Analysis

| | | | | |
|---|---|---|---|---|
| $C_{23}H_{29}N_5O_9$ | Found % | C 53.48 | H 5.61 | N 13.22 |
| | CALC. % | C 53.17 | H 5.63 | N 13.48 |

N-Isopropyl-N'-/¯4-(5-N,N-dimethylamino methyl-oxazol-5-yl-phenyl_7 formamidine (Compound 68)

Maleate salt (ethylacetate) M.p. 160-162°C (dec.)

Analysis

| | | | | |
|---|---|---|---|---|
| $C_{24}H_{30}N_4O_9$ | Found % | C 55.91 | H 5.95 | N 10.83 |
| | Calc. % | C 55.59 | H 5.83 | N 10.81 |

N-Isopropyl-N'-/‾3-(5-pyrrolidino methyl-1.2.4-oxadiazol-3-yl)-phenyl_/ formamidine (Compound 69)

Citrate salt (ethylacetate) M.p. 65-70°C (dec.)

Analysis

| | | | | |
|---|---|---|---|---|
| $C_{29}H_{39}N_5O_{15}$ | Found % | C 49.61 | H 5.77 | N 10.19 |
| | Calc. % | C 49.92 | H 5.63 | N 10.04 |

N-Ethyl-N'-/‾3-(5-pyrrolidino methyl-1.2.4-oxadiazol-3-yl)-phenyl_/ formamidine (Compound 70)

Tartrate salt (ethylacetate-acetone) M.p. 80-85°C (dec.)

Analysis

| | | | | |
|---|---|---|---|---|
| $C_{24}H_{33}N_5O_{13}$ | Found % | C 48.39 | H 5.41 | N 11.87 |
| | Calc. % | C 48.08 | H 5.55 | N 11.68 |

N-Isopropyl-N'-/‾3-(2-N,N-diethylamino methyl-oxazol-5-yl)-phenyl_/ formamidine (Compound 71)

Tartrate salt (ethylacetate) M.p. 160-165°C

Analysis

| | | | | |
|---|---|---|---|---|
| $C_{22}H_{32}N_4O_7$ | Found % | C 56.55 | H 7.02 | N 12.12 |
| | Calc. % | C 56.88 | H 6.94 | N 12.06 |

N-(2-Methoxy ethyl)-N'-/¯4-(pyrimidin-4-yl)-phenyl_7 formamidine

(Compound 72)

Fumarate salt (ethanol) M.p. 160-162°C (dec.)

Analysis

| | | | | |
|---|---|---|---|---|
| $C_{40}H_{44}N_8O_{14}$ | Found % | C 55.70 | H 5.22 | N 13.18 |
| | Calc. % | C 55.81 | H 5.15 | N 13.02 |

N-Isopropyl-N'-/¯3-(2-methyl-oxazol-5-yl)-phenyl_7 formamidine

(Compound 73)

Nitrate salt (acetone-ethylacetate) M.p. 96-98°C (dec.)

Analysis

| | | | | |
|---|---|---|---|---|
| $C_{14}H_{18}N_4O_4$ | Found % | C 54.37 | H 6.01 | N 18.45 |
| | Calc. % | C 54.89 | H 5.92 | N 18.29 |

N-Methyl-N'-/¯4-(2-dimethylamino methyl-4-thiazolyl)-phenyl_7 formamidine (Compound 92)

Maleate salt (acetone) M.p. 133-136°C (dec.)

Analysis

| | | | | |
|---|---|---|---|---|
| $C_{22}H_{26}N_4O_8S$ | Found % | C 52.15 | H 4.95 | N 11.00 |
| | Calc. % | C 52.17 | H 5.17 | N 11.06 |

N-Isopropyl-N'-/‾3-(2-dimethylamino methyl-4-thiazolyl)-phenyl_7 formamidine (Compound 100)

Maleate salt (ethanol) M.p. 147-149°C (dec.)

Analysis

| | | | | |
|---|---|---|---|---|
| $C_{24}H_{30}N_4O_8S$ | Found % | C 53.92 | H 5.63 | N 10.41 |
| | Calc. % | C 53.92 | H 5.66 | N 10.48 |

N-Methyl-N'-/‾3-(2-amino-4-thiazolyl)-phenyl_7 formamidine (Compound 103)

Maleate salt (ethanol) M.p. 168-169°C (dec.)

Analysis

| | | | | |
|---|---|---|---|---|
| $C_{19}H_{20}N_4O_8S$ | Found % | C 49.18 | H 4.25 | N 11.74 |
| | Calc. % | C 49.14 | H 4.34 | N 12.06 |

N-Isopropyl-N'-/‾3-(2-amino-4-thiazolyl)-phenyl_7 formamidine (Compound 104)

Maleate salt (ethanol) M.p. 162-163°C (dec.)

Analysis

| | | | | |
|---|---|---|---|---|
| $C_{21}H_{24}N_4O_8S$ | Found % | C 50.95 | H 4.85 | N 11.32 |
| | Calc. % | C 51.21 | H 4.91 | N 11.37 |

N-Isopropyl-N'-/‾3-(2-methylamino-4-thiazolyl)-phenyl_7 formamidine

(Compound 105)

Maleate salt (ethanol

Analysis

| | | | | |
|---|---|---|---|---|
| $C_{22}H_{26}N_4O_8S$ | Found % | C 52.45 | H 5.24 | N 10.97 |
| | Calc. % | C 52.17 | H 5.18 | N 11.06 |

N-Isopropyl-N'-/‾4-(2-methylamino-4-thiazolyl)-phenyl_7 formamidine

(Compound 106)

Maleate salt (ethanol) M.p. 162-163°C (dec.)

Analysis

| | | | | |
|---|---|---|---|---|
| $C_{22}H_{26}N_4O_8S$ | Found % | C 52.39 | H 5.17 | N 11.20 |
| | Calc. % | C 52.17 | H 5.18 | N 11.06 |

Example 23

N-/‾(4-Hydroxymethyl-1.3-dioxolan-2-yl)-methyl_7-N'-/‾4-(imidazol--4-yl)-phenyl_7 formamidine (Compound 74)

A suspension of N-cyano-N'-/‾4-(imidazol-4-yl)-phenyl_7 formamidine (1.17 g) and 4-hydroxymethyl-2-aminomethyl-1.3-dioxolane (3.7 g) in ethanol (25 ml) and water (0.3 ml) was stirred until a clear solution resulted. Ethanol (20 ml) was added and the solution was

filtered on charcoal. A solution of maleic acid (3.8 g) in ethanol was slowly added. The solid which crystallized out was filtered, and dried to give 1.9 g of the title compound.

Maleate salt (ethanol) M.p. 139-141°C (dec.)

Analysis

| | | | | |
|---|---|---|---|---|
| $C_{23}H_{26}N_4O_{11}$ | Found % | C 51.96 | H 4.94 | N 10.39 |
| | Calc. % | C 51.68 | H 4.90 | N 10.48 |

By utilizing suitable N-cyano-formamidine derivatives and the amines the following compounds were prepared:

__N-(1-Methyl-2-hydroxyethyl)-N'-[4-(imidazol-4-yl)-phenyl] formamidine__ __(Compound 75)__

Maleate salt (acetone) M.p. 160°C (dec.)

Analysis

| | | | | |
|---|---|---|---|---|
| $C_{21}H_{24}N_4O_9$ | Found % | C 53.06 | H 5.12 | N 11.72 |
| | Calc. % | C 52.94 | H 5.08 | N 11.76 |

__N-(2-Ethoxymethyl thio-ethyl)-N'-[4(imidazol-4-yl)-phenyl] formamidine__ __(Compound 76)__

Maleate salt (acetone) M.p. 139-140°C (dec.)

Analysis

| | | | | |
|---|---|---|---|---|
| $C_{23}H_{28}N_4O_9S$ | Found % | C 51.40 | H 5.25 | N 10.43 |
| | Calc. % | C 51.48 | H 5.26 | N 10.44 |

N-n-Propyl-N'-/‾2-bromo-4-(imidazol-4-yl)-phenyl_7 formamidine (Compound 77)

Maleate salt (acetone) M.p. 162-163°C

Analysis

| | | | | | | | |
|---|---|---|---|---|---|---|---|
| $C_{21}H_{23}BrN_4O_8$ | Found % | C | 47.08 | H | 4.27 | N | 10.35 |
| | Calc. % | C | 46.76 | H | 4.29 | N | 10.39 |

N-neo-Pentyl-N'-/‾2-bromo-4-(imidazol-4-yl)-phenyl_7 formamidine (Compound 78)

Maleate salt (acetone) M.p. 151-154°C

Analysis

| | | | | | | | |
|---|---|---|---|---|---|---|---|
| $C_{23}H_{27}BrN_4O_8$ | Found % | C | 48.06 | H | 4.73 | N | 9.69 |
| | Calc. % | C | 48.68 | H | 4.79 | N | 9.87 |

N-(2-Methyl thio-ethyl)-N'-/‾2-bromo-4-(imidazol-4-yl)-phenyl_7 formamidine (Compound 79)

Maleate salt (acetone) M.p. 150-153°C (dec.)

Analysis

| | | | | | | | |
|---|---|---|---|---|---|---|---|
| $C_{21}H_{23}BrN_4O_8S$ | Found % | C | 44.36 | H | 4.15 | N | 9.81 |
| | Calc. % | C | 44.14 | H | 4.06 | N | 9.81 |

N-n-Propyl-N'-/¯4-(pyrimidyn-4-one-5-yl)-phenyl_7 formamidine

(Compound 80)

Maleate salt (acetone) M.p. 146-149°C (dec.)

Analysis

| | | | | |
|---|---|---|---|---|
| $C_{22}H_{24}N_4O_9$ | Found % | C 54.57 | H 4.97 | N 11.86 |
| | Calc. % | C 54.09 | H 4.95 | N 11.47 |

N-neo-Pentyl-N'-/¯2-chloro-4-(imidazol-4-yl)-phenyl_7 formamidine

(Compound 81)

Maleate salt (acetone) M.p. 180-181°C

Analysis

| | | | | |
|---|---|---|---|---|
| $C_{23}H_{27}ClN_4O_8$ | Found % | C 52.47 | H 5.28 | N 10.61 |
| | Calc. % | C 52.82 | H 5.20 | N 10.71 |

N-n-Propyl-N'-/¯2.6-dichloro-4-(imidazol-4-yl)-phenyl_7 formamidine

(Compound 82)

Maleate salt (acetone) M.p. 143-145°C (dec.)

Analysis

| | | | | |
|---|---|---|---|---|
| $C_{21}H_{22}Cl_2N_4O_8$ | Found % | C 47.73 | H 4.09 | N 10.65 |
| | Calc. % | C 47.65 | H 4.19 | N 10.58 |

N-Allyl-N'-/ 2-chloro-6-bromo-4-(imidazol-4-yl)-phenyl_7 formamidine (Compound 83)

Maleate salt (acetone) M.p. 148-151°C (dec.)

Analysis

| | | | | |
|---|---|---|---|---|
| $C_{21}H_{20}BrClN_4O_8$ | Found % | C 43.98 | H 3.64 | N 9.69 |
| | Calc. % | C 44.11 | H 3.52 | N 9.80 |

N-neo-Pentyl-N'-/ 4-(1.2.4-triazol-3-yl)-phenyl_7 formamidine (Compound 89)

Maleate salt (acetone) M.p. 144-146°C (dec.)

Analysis

| | | | | |
|---|---|---|---|---|
| $C_{22}H_{27}N_5O_8$ | Found % | C 54.17 | H 5.62 | N 14.41 |
| | Calc. % | C 53.98 | H 5.56 | N 14.31 |

Example 24

N-Phenyl-N'-/ 3-/ 5-(pyrrolidino methyl)-thiophen-2-yl_7-phenyl_7 formamidine (Compound 84)

A suspension of 2-pyrrolidino methyl-5-(3-aminophenyl)-thiophene (0.5 g) and ethyl-N-phenyl formimidate (0.6 g) in ethanol (10 ml) was stirred 3 days at room temperature and filtered. The solid was dried to give the title compound, M.p. 145-146°C.

Analysis

| | | | | |
|---|---|---|---|---|
| $C_{22}H_{23}N_3S$ | Found % | C 70.33 | H 6.55 | N 11.49 |
| | Calc. % | C 70.09 | H 6.41 | N 11.63 |

N-Phenyl-N'-/¯4-(pyridin-2-yl)-phenyl_/ formamidine (Compound 85) was similarly prepared, M.p. 115-118°C.

Analysis

| | | | | |
|---|---|---|---|---|
| $C_{18}H_{15}N_3$ | Found % | C 79.08 | H 5.52 | N 15.30 |
| | Calc. % | C 79.09 | H 5.53 | N 15.38 |

The following not limitative examples of pharmaceutical compositions according to the invention are reported:

Example 25

Tablets

| | |
|---|---|
| - active ingredient | 50 mg |
| - lactose | 217 mg |
| - corn starch | 30 mg |
| - magnesium stearate | 3 mg |

Method of preparation: the active ingredient, lactose, and corn starch were mixed and homogeneously moistened with water. After screening of the most mass and drying in a tray driver, the mixture was again passed through a screen and magnesium stearate was added. Then the mixture was pressed into tablets weighing 300 mg each. Each tablet contains 50 mg of active ingredient.

Example 26

Capsules

- active ingredient 50 mg
- corn starch 170 mg
- magnesium stearate 2 mg

Method of preparation: the active ingredient was mixed with the auxiliary products, and the mixture was passed through a screen and mixed homogeneously in a suitable device. The resulting mixture was filled into hard gelatine capsule (222 mg per capsule); each capsule contains 50 mg of active ingredient.

Example 27

Vials

- active ingredient 50 mg
- water for injection q.s. 5 ml

Method of preparation: the active ingredient was dissolved in the water in appropriate amounts, and then the resulting solution was introduced into 5 ml vials under sterile conditions. Each vial contains 50 mg of active ingredient.

Claims

1. Compounds of general formula (I)

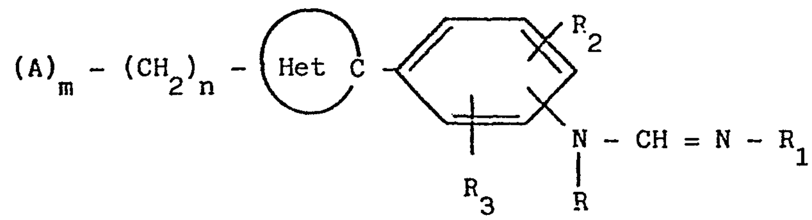

(I)

wherein A represents a hydrogen atom, an amino group which may be substituted by one or two alkyl groups having 1 to 3 carbon atoms or a saturated heterocyclic ring, R represents a hydrogen atom or a methyl group, $R_1$ represents a linear or branched alkyl group which may contain 1 or 2 heteroatoms such as a sulfur, an oxygen or nitrogen atom or may form a substituted or an unsubstituted cyclic ring, an alkenyl group, an alkynyl group, a cycloalkyl group or a phenyl group, $R_2$ and $R_3$ which may be the same or different, represent a hydrogen atom, an alkyl or alkoxy group having 1 to 3 carbon atoms or a halogen atom; m and n represent 0 or 1, Het represents a substituted or unsubstituted heterocyclic ring containing from 1 to 3 heteroatoms (except the imidazole ring when $R_1$ is a linear or branched alkyl group not containing heteroatoms and $R_2$ and $R_3$ are both hydrogen atoms), tautomers thereof and acid addition salts of the aforesaid compounds.

2. Physiologically compatible acid addition salts of compounds of formula I as claimed in claim 1.

3. Salts as claimed in claim 2 formed with maleic, hydrochloric, fumaric or sulphuric acid.

4. Compounds as claimed in any one of the preceding claims wherein the formamidine radical is in the para position of the benzene ring with respect to the Het group, and m and n are 0.

5. Compounds as claimed in any one of the preceding claims wherein R is a hydrogen atom, $R_1$ is propyl, isopropyl, butyl, sec-butyl, isobutyl, neopentyl, allyl, methoxyethyl, dimethoxy-ethyl, hydroxypropyl, hydroxybutyl, methylthioethyl, ethylthio-ethyl, cyanoethyl or ethoxymethylthioethyl group, $R_2$ and/or $R_3$ represent a hydrogen atom, a methyl, methoxy group, a bromine, chlorine or fluorine atom, Het is 3-pyrazolyl, 3-(1,2,4--triazolyl) or 4-imidazolyl group.

6. N-(2-Methoxy ethyl)-N'-[4-(imidazol-4-yl)-phenyl] formamidine

7. N-(2-hydroxy propyl)-N'-[4-(imidazol-4-yl)-phenyl] formamidine

8. N-(2-Methyl thio ethyl)-N'-[4-(imidazol-4-yl)-phenyl] formamidine

9. N-(2-Ethoxymethyl thio ethyl)-N'-[4-(imidazol-4-yl)-phenyl] formamidine

10. N-(2-Cyano ethyl)-N'-/̄4-(imidazol-4-yl)-phenyl/̲ formamidine

11. N-n-Butyl-N'-/̄4-(imidazol-4-yl)-2-methyl-phenyl/̲ formamidine

12. N-Allyl-N'-/̄4-(imidazol-4-yl)-2-methyl-phenyl/̲ formamidine

13. N-Propyl-N'-/̄4-(imidazol-4-yl)-2-methoxy-phenyl/̲ formamidine

14. N-Isopropyl-N'-/̄4-(pyrazol-3-yl)-phenyl/̲ formamidine

15. N-Propyl-N'-/̄4-(imidazol-4-yl)-2-bromo-phenyl/̲ formamidine

16. N-Isopropyl-N'-/̄4-(1.2.4-triazol-3-yl)-phenyl/̲ formamidine

17. Physiologically compatible acid addition salts of compounds as claimed in claims 6 to 16.

18. Maleic, hydrochloric, fumaric or sulphuric acid addition salts of compounds as claimed in claims 6 to 16.

19. A process for the preparation of compounds of general formula (I) as claimed in claim 1 which comprises reacting a compound of formula (II)

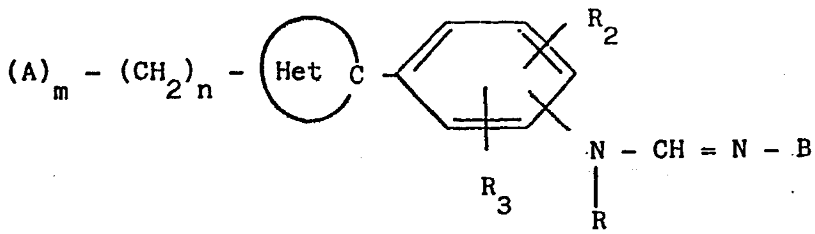

(II)

in which R, $R_2$, $R_3$, A, Het, m and n are as defined in claim 1 and B represents a suitable leaving group, with an amine of formula (III)

$H_2N - R_1$ (III)

in which $R_1$ is as defined in claim 1.

20. A process as claimed in claim 19 in which B is a cyano group.

21. A process as claimed in claims 19 and 20 in which the reaction is carried out in the presence of water or of an inert aqueous organic solvent.

22. A process as claimed in claim 21 in which the inert organic solvent includes dimethylformamide or dioxane.

23. A process as claimed in claims 19 to 22 in which the reaction is performed at room temperature.

24. A process for the preparation of compounds of general formula (I) as claimed in claim 1 wherein $R_1$ represents a phenyl group, which comprises reacting an amine of formula (IV)

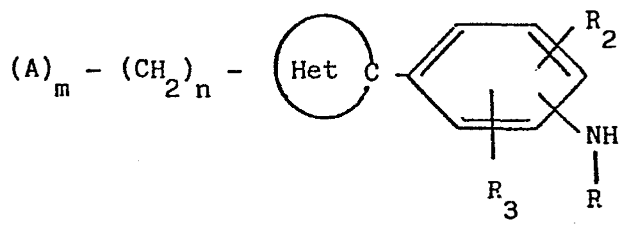

(IV)

(wherein R, $R_2$, $R_3$, A, Het, m and n are as defined in claim 1) with a N-phenyl alkylimidate of formula (VII)

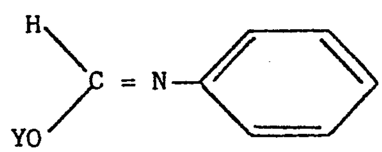

(VII)

in which Y is methyl or ethyl.

25. A process as claimed in claim 24 in which the reaction is carried out in the presence of an inert organic solvent.

26. A process as claimed in claim 25 in which the inert organic solvent comprises ethanol, dioxane or acetone.

27. A process as claimed in claims 24 to 26 in which the reaction is effected at room temperature.

28. Pharmaceutical compositions comprising as active ingredient at least one compound of general formula (I) as defined in claim 1 or a tautomer or physiologically compatible acid addition salt thereof, in association with a pharmaceutical carrier or excipient.

29. Pharmaceutical compositions as claimed in claim 28 for use as anti-ulcers.

30. Pharmaceutical compositions as claimed in claim 28 for treatment of patients suffering from disorders of gastrointestinal tract.

31. Method of treatment of patients suffering from ulcers by means of compositions according to anyone of the claims 28 to 30.

32. New intermediate compounds of formula II

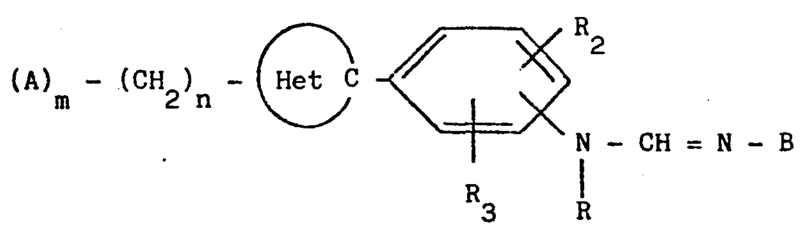

in which R, $R_2$, $R_3$, A, Het, m and n are as defined in claim 1 and B is as defined in claim 19 for the performance of the process as claimed in claim 19.

for Austria
für Österreich
pour l'Autriche

CLAIMS FOR AUSTRIA :

1. A process for the preparation of compounds of general formula (I)

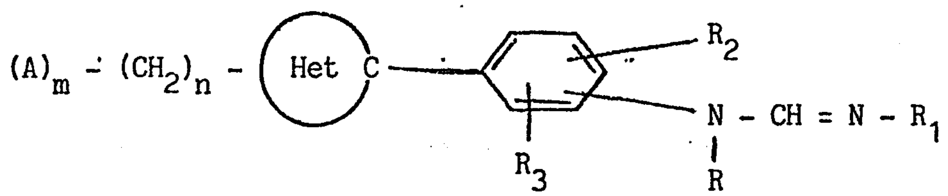

(I)

wherein A represents a hydrogen atom, an amino group which may be substituted by one or two alkyl groups having 1 to 3 carbon atoms or a saturated heterocyclic ring, R represents a hydrogen atom or a methyl group, $R_1$ represents a linear or branched alkyl group which may contain 1 or 2 heteroatoms such as a sulfur, an oxygen or nitrogen atom or may form a substituted or an unsubstituted cyclic ring, an alkenyl group, an alkynyl group, a cycloalkyl group or a phenyl group, $R_2$ and $R_3$ which may be the same or different, represent a hydrogen atom, an alkyl or alkoxy group having 1 to 3 carbon atoms or a halogen atom, m and n represent 0 or 1, Het represents a substituted or unsubstituted heterocyclic ring containing from 1 to 3 heteroatoms (except the imidazole ring when $R_1$ is a linear or branched alkyl group not containing heteroatoms and $R_2$ and $R_3$ are both hydrogen atoms), tautomers thereof and acid addition salts of the aforesaid compounds which comprises reacting a compound of formula (II)

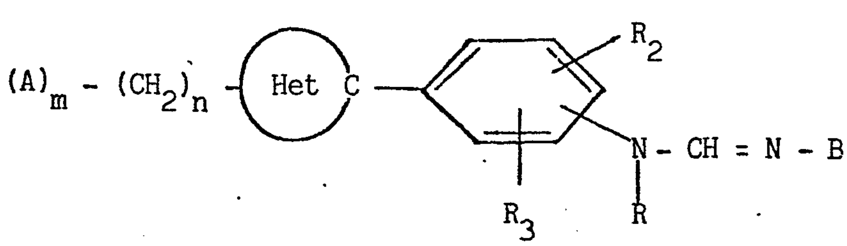

(II)

in which R, $R_2$, $R_3$, A, Het, m and n are as defined above and B represents a suitable leaving group, with an amine of formula (III)

for Austria
für Österreich
pour l'Autriche

$$H_2N - R_1 \qquad (III)$$

in which $R_1$ is as defined above.

2. A process as claimed in Claim 1 in which B is a cyano group.

3. A process as claimed in Claims 1 and 2 in which the reaction is carried out in the presence of water or of an inert aqueous organic solvent.

4. A process as claimed in Claim 3 in which the inert organic solvent includes dimethylformamide or dioxane.

5. A process as claimed in Claims 1 to 4 in which the reaction is performed at room temperature.

6. A process for the preparation of compounds of general formula (I) as claimed in Claim 1 wherein $R_1$ represents a phenyl group, which comprises reacting an amine of formula (IV)

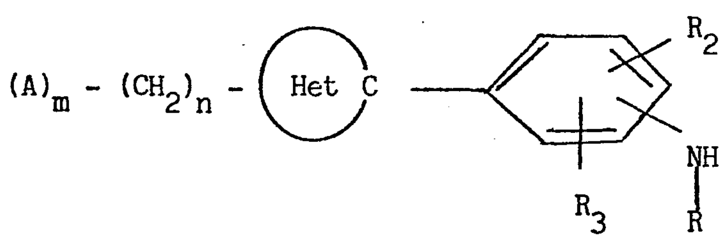

(IV)

(wherein R, $R_2$, $R_3$, A, Het, m and n are as defined in Claim 1) with a N-phenyl alkylimidate of formula (VII)

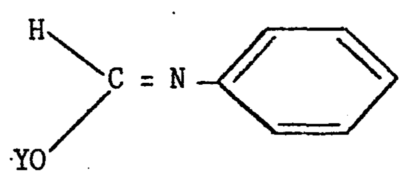

(VII)

in which Y is methyl or ethyl.

7. A process as claimed in Claim 6 in which the reaction is carried out in the presence of an inert organic solvent.

8. A process as claimed in Claim 7 in which the inert organic solvent comprises ethanol, dioxane or acetone.

for Austria
für Österreich
pour l'Autriche

9. A process as claimed in Claims 6 to 8 in which the reaction is effected at room temperature.